# EUROPÄISCHE PATENTSCHRIFT

(11) **EP 0 885 187 B1**
(45) Veröffentlichungstag und Bekanntmachung des Hinweises auf die Patenterteilung: **26.02.2003**
(21) Anmeldenummer: 97914186.8
(22) Anmeldetag: 04.03.1997
(51) Int. Cl.: C07C 251/48, C07C 251/44, C07C 259/04, A01N 37/50, A01N 37/38, A01N 47/24

(54) **DIPHENYLETHER, VERFAHREN UND ZWISCHENPRODUKTE ZU IHRER HERSTELLUNG UND IHRE VERWENDUNG**
DIPHENYLETHERS, PROCESS AND INTERMEDIATE PRODUCTS FOR THEIR PREPARATION, AND THEIR USE
DIPHENYLETHERS, PROCEDE ET PRODUITS INTERMEDIAIRES POUR LEUR PREPARATION, ET LEUR UTILISATION

(30) Priorität: 08.03.1996 DE 19609037; 13.03.1996 DE 19609768
(43) Veröffentlichungstag der Anmeldung: 23.12.1998
(73) Patentinhaber: BASF AKTIENGESELLSCHAFT, 67056 Ludwigshafen (DE)
(72) Erfinder: OBERDORF, Klaus, D-69117 Heidelberg (DE); GRAMMENOS, Wassilios, D-67063 Ludwigshafen (DE); SAUTER, Hubert, D-68167 Mannheim (DE); GROTE, Thomas, D-67105 Schifferstadt (DE); MÜLLER, Bernd, D-67227 Frankenthal (DE); KIRSTGEN, Reinhard, D-67434 Neustadt (DE); BAYER, Herbert, D-68159 Mannheim (DE); PTOCK, Arne, D-67067 Ludwigshafen (DE); RACK, Michael, D-69123 Heidelberg (DE); HARREUS, Albrecht, D-67063 Ludwishafen (DE); RÖHL, Franz, D-67105 Schifferstadt (DE); AMMERMANN, Eberhard, D-64646 Heppenheim (DE); HARRIES, Volker, D-67227 Frankenthal (DE); LORENZ, Gisela, D-67434 Hambach (DE); STRATHMANN, Siegfried, D-67117 Limburgerhof (DE)
(86) Internationale Anmeldenummer: EP9701079
(87) Internationale Veröffentlichungsnummer: WO97032842

(56) Entgegenhaltungen:
- EP-A- 0 307 103
- EP-A- 0 398 692
- EP-A- 0 513 580
- EP-A- 0 673 923
- WO-A-93/15046
- CHEMICAL ABSTRACTS, vol. 60, no. 9, 27.April 1964 Columbus, Ohio, US; abstract no. 10731a, XP002034237 & CHEM. PHARM. BULL. (TOKYO), Bd. 11, Nr. 10, 1963, Seiten 1299-1305, T. KAMETANI ET AL:

## Beschreibung

Die vorliegende Erfindung betrifft Diphenylether der Formel I, 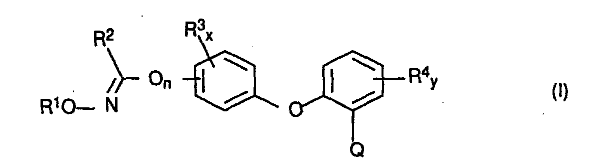 sowie deren Salze und N-Oxide, in denen die Substituenten und Indices die folgende Bedeutung haben:
- Q: C(CO₂CH₃)=NOCH₃ oder C(CONHCH₃)=NOCH₃
- n: 0 oder 1;
- R¹: Wasserstoff oder
ein über ein Kohlenstoff-Atom gebundener organischer Rest;
- R²: Wasserstoff, Cyano, Halogen oder
ein über ein Kohlenstoff-, Sauerstoff-, Schwefel- oder Stickstoff-Atom gebundener organischer Rest, oder
für den Fall, daß n für 1 steht, ein über ein Kohlenstoff-Atom gebundener organischer Rest;
- x: 0, 1 oder 2, wobei die Reste R³ verschieden sein können, wenn x für 2 steht;
- R³: Cyano, Nitro, Halogen oder
ein überein Kohlenstoff-, Sauerstoff-, Schwefel- oder Stickstoff-Atom gebundener organischer Rest oder
- R²: und ein zur Gruppe Oₙ-C(R²)=NOR¹ benachbarter Substituent R³ C₂-C₃-Alkylen, welches durch ein Sauerstoff- oder Schwefelatom unterbrochen sein kann oder über ein Sauerstoff- oder Schwefelatom an den Phenylring gebunden sein kann;
- y: 0, 1, 2 oder 3, wobei die Reste R⁴ verschieden sein können, wenn y für 2 oder 3 steht;
- R⁴: Cyano, Halogen, C₁-C₄-Alkyl, C₁-C₄-Halogenalkyl oder C₁-C₄-Alkoxy.

Desweiteren betrifft die Erfindung Verfahren und Zwischenprodukte zur Herstellung dieser Verbindungen sowie ihre Verwendung zur Bekämpfung von tierischen Schädlingen und Schadpilzen.

Aus der Literatur (EP-A 253 213; EP-A 254 426; EP-A 280 185; EP-A 307 103; EP-A 398 692; EP-A 477 631; EP-A 513 580; WO-A 93/15,046) sind Phenylether mit fungiziden bzw. fungiziden und insektiziden Eigenschaften bekannt, die sich von den erfindungsgemäßen Verbindungen durch die Substituenten unterscheiden.

Demgegenüber lagen der vorliegenden Erfindung Verbindungen mit verbesserter Wirkung und verbreitertem Wirkungsspektrum als Aufgabe zugrunde.

Demgemäß wurden die eingangs definierten Verbindungen I gefunden. Desweiteren wurden Verfahren zur Herstellung dieser Verbindungen sowie ihre Verwendung zur Bekämpfung von tierischen Schädlingen und Schadpilzen gefunden.

Die Verbindungen I sind auf verschiedenen Wegen nach an sich in der Literatur beschriebenen Verfahren erhältlich.

Der Aufbau der Gruppierung Q ist beispielsweise aus der eingangs zitierten Literatur bekannt und erfolgt im allgemeinen und im besonderen nach den dort beschriebenen Verfahren.

Üblicherweise geht man bei der Synthese der Verbindungen I, in denen n für 0 steht, so vor, daß man ein Phenol der Formel IIa in einem inerten Lösungsmittel mit einem Halogenbenzol der Formel IIIa in den entsprechenden Ether der Formel IVa überführt und IVa anschließend mit einem O-substituierten Hydroxylamin (R¹-O-NH₂) oder dessen Salz zu I umsetzt.

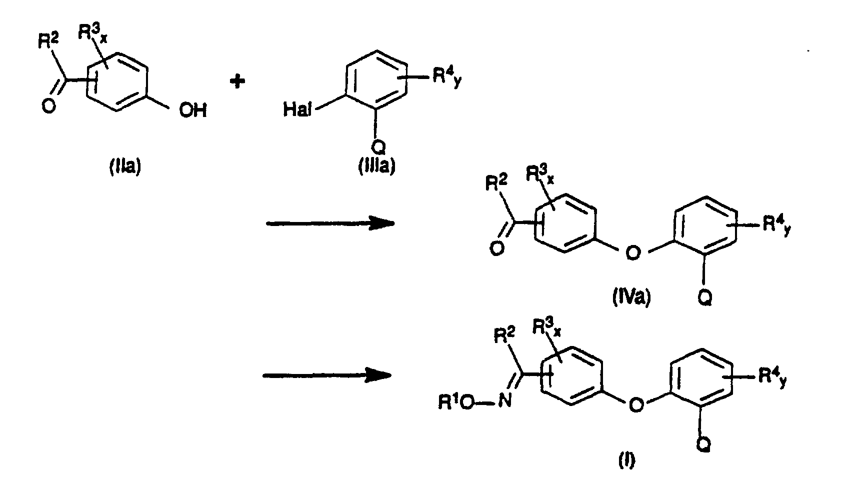

Hal in der Formel IIIa steht für ein Halogenatom wie Fluor, Chlor, Brom und Iod, vorzugsweise Fluor, Chlor oder Brom.
1a) Die Umsetzung von IIa mit IIIa erfolgt üblicherweise in einem inerten Lösungsmittel bei Temperaturen von 0°C bis 130°C, vorzugsweise 20°C bis 80°C in Gegenwart einer Base.
   Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, sowie Dimethylsulfoxid und Dimethylformamid, besonders bevorzugt Tetrahydrofuran, Acetonitril, Dimethylsulfoxid und Aceton. Es können auch Gemische der genannten Lösungsmittel verwendet werden.
   Als Basen kommen allgemein anorganische Verbindungen wie Alkalimetall- und Erdalkalimetallhydroxide wie Lithiumhydroxid, Natriumhydroxid, Kaliumhydroxid und Calziumhydroxid, Alkalimetall- und Erdalkalimetalloxide wie Lithiumoxid, Natriumoxid, Calziumoxid und Magnesiumoxid, Alkalimetallund Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat und Calziumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium- tert.-Butanolat und Dimethoxymagnesium, außerdem organische Basen, z.B. tertiäre Amine wie Tri-methylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Kaliumcarbonat, Natriumhydrid und Kalium-tert.-butylat. Die Basen werden im allgemeinen in katalytischen Mengen eingesetzt, sie können aber auch äquimolar, im Überschuß oder gegebenenfalls als Lösungsmittel verwendet werden.
   Die Edukte werden im allgemeinen in äquimolaren Mengen miteinander umgesetzt. Es kann für die Ausbeute vorteilhaft sein, IIa in einem Überschuß bezogen auf IIIa einzusetzen.
1b) Die Umsetzung von IVa mit dem O-substituierten Hydroxylamin oder dessen Salz erfolgt üblicherweise in einem inerten Lösungsmittel bei Temperaturen von 0°C bis 80°C, vorzugsweise 20°C bis 60°C, ggf. in Gegenwart einer Säure oder ggf. in Gegenwart einer Base, wenn das O-substituierte Hydroxylamin aus seinem Salz freigesetzt wird.
   Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Alkohole wie Methanol, Ethanol, n-Propanol, Isopropanol, n-Butanol und tert.-Butanol sowie Dimethylsulfoxid, Dimethylformamid und Pyridin, besonders bevorzugt Methanol und Pyridin. Es können auch Gemische der genannten Lösungsmittel verwendet werden.
   Als Basen kommen allgemein anorganische Verbindungen wie Alkalimetall- und Erdalkalimetallhydroxide wie Lithiumhydroxid, Natriumhydroxid, Kaliumhydroxid und Calziumhydroxid, Alkalimetall- und Erdalkalimetalloxide wie Lithiumoxid, Natriumoxid, Calziumoxid und Magnesiumoxid, Alkalimetallund Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat und Calziumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium- tert.-Butanolat und Dimethoxymagnesium, außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Pyridin und Natriumhydroxid. Die Basen werden im allgemeinen in katalytischen Mengen eingesetzt, sie können aber auch äquimolar, im Überschuß oder gegebenenfalls als Lösungsmittel verwendet werden.
   Als Säuren und saure Katalysatoren finden anorganische Säuren wie Fluorwasserstoffsäure, Salzsäure, Bromwasserstoffsäure, Schwefelsäure und Perchlorsäure, Lewis-Säuren wie Bortrifluorid, Aluminiumtrichlorid, Eisen-III-chlorid, Zinn-IV-chlorid, Titan-IV-chlorid und Zink-II-chlorid, sowie organische Säuren wie Ameisensäure, Essigsäure, Propionsäure, Oxalsäure, Zitronensäure und Trifluoressigsäure Verwendung. Die Säuren werden im allgemeinen in katalytischen Mengen eingesetzt.
   Die Edukte werden im allgemeinen in äquimolaren Mengen miteinander umgesetzt. Es kann für die Ausbeute vorteilhaft sein, das Hydroxylamin bzw. dessen Salz in einem Überschuß bezogen auf IVa einzusetzen.

In entsprechender Weise erhält man die Verbindungen I, in denen n für 0 steht, dadurch, daß man ein Phenol der Formel IIa zunächst mit einem O-substituierten Hydroxylamin (R¹-O-NH₂) oder dessen Salz in die entsprechende Verbindung der Formel IIb überführt und IIb anschließend in einem inerten Lösungsmittel mit einem Halogenbenzol der Formel IIIa zu I umsetzt.

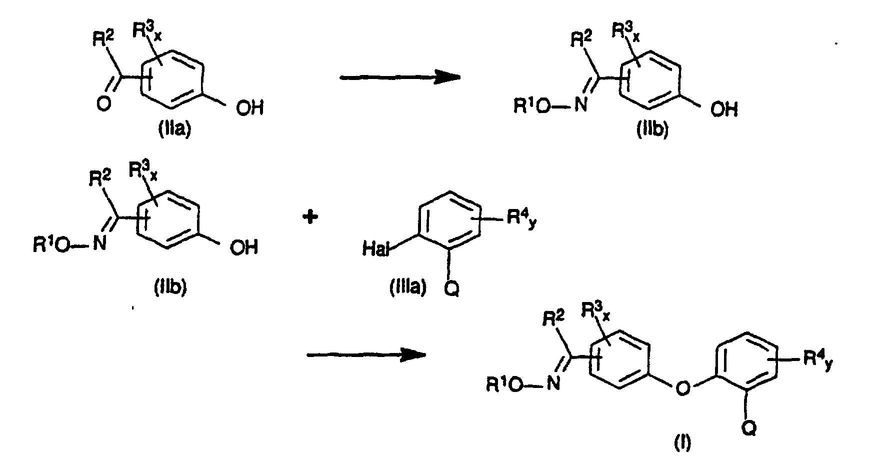

Die Umsetzungen erfolgen im allgemeinen und im besonderen nach den vorstehend beschriebenen Methoden.

Die Ausgangsstoffe der Formel IIa, in denen n für 0 steht, können erhalten werden, indem man ein geeignet substituiertes Phenol der Formel IIc in einem inerten Lösungsmittel in Gegenwart einer metallorganischen Base mit einer aktivierten Carbonsäure der Formel Va umsetzt [vgl. J. Organomet. Chem. 56, 53-66 (1973); Chem. Ber. 125, 1169-1190 (1992)].

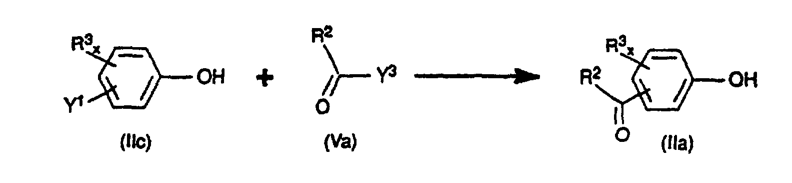

Y¹ in der Formel IIc steht für ein Halogenatom, z.B. Fluor, Chlor, Brom und Iod, besonders Brom und Iod.

Y³ in der Formel Va steht für ein Halogenatom, z.B. Fluor, Chlor, Brom und Iod, besonders Chlor, oder einen Amid- oder einen Esterrest. Anstelle der Verbindung Va kann auch ein entsprechendes Cyanid R²-C≡N eingesetzt werden.

Diese Umsetzung erfolgt üblicherweise in einem inerten Lösungsmittel in Gegenwart einer metallorganischen Basebei Temperaturen von -75°C bis 40°C, vorzugsweise -75°C bis 0°C.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, Ether wie Diethylether, Diisopropylether, tert.-Butylmethylether, Dioxan, Anisol und Tetrahydrofuran, sowie Dimethylsulfoxid und Dimethylformamid, besonders bevorzugt Diethylether und Tetrahydrofuran. Es können auch Gemische der genannten Lösungsmittel verwendet werden.

Als metallorganische Basen kommen allgemein metallorganische Verbindungen, insbesondere Alkalimetallalkyle wie Methyllithium, Butyllithium und Phenyllithium, Alkylmagnesiumhalogenide wie Methylmagnesiumchlorid sowie in Betracht. Besonders bevorzugt werden n-Butyllithium. Die Basen können im allgemeinen äquimolar oder im Überschuß verwendet werden.

Die Edukte werden im allgemeinen in äquimolaren Mengen miteinander umgesetzt. Es kann für die Ausbeute vorteilhaft sein, Va in einem Überschuß bezogen auf IIc einzusetzen.

Nach einer weiteren Methode erhält man die Verbindungen IIa auch dadurch, daß man ein Hydroxybenzoesäure-halogenid der allgemeinen Formel IId in einem inerten Lösungsmittel mit einer metallorganischen Verbindung (R²-M; M steht für das Äquivalent eines Metallions) umsetzt [vgl. DE-A 38 38 243; EP-A 446 872].

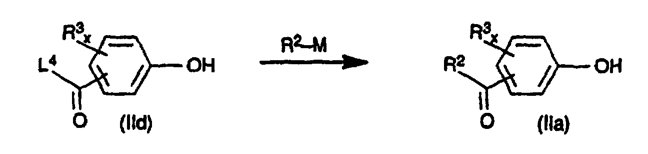

Als Metall eignen sich besonders Litium, Magnesium, Kupfer und Zink.

L⁴ in der Formel IId steht für ein Halogenatom, z.B. Fluor, Chlor, Brom und Iod, besonders Chlor.

Diese Umsetzung erfolgt üblicherweise in einem inerten Lösungsmittel bei Temperaturen von -80°C bis 20°C, vorzugsweise -75°C bis 0°C.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, Ether wie Diethylether, Diisopropylether, tert.-Butylmethylether, Dioxan, Anisol und Tetrahydrofuran, besonders bevorzugt Diethylether und Tetrahydrofuran. Es können auch Gemische der genannten Lösungsmittel verwendet werden.

Die Edukte werden im allgemeinen in äquimolaren Mengen miteinander umgesetzt. Es kann für die Ausbeute vorteilhaft sein, die metallorganische Verbindung in einem Überschuß bezogen auf IId einzusetzen.

Die Ausgangsstoffe der Formel IIa erhält man außerdem dadurch, daß man ein geeignet substituiertes Phenzol der Formel IIe in Gegenwart einer Base mit einem Alkylhalogenid (R'-Hal; R' bedeutet C₁-C₄-Alkyl, Hal steht für ein Halogenatom wie Fluor, Chlor, Brom und Iod, vorzugsweise Chlor und Brom) in den entsprechenden Alkyl-phenylether der Formel IIf überfüht, IIf anschließend in Analogie zum vorstehend beschriebenen Verfahren (Umsetzung von IIc) durch Umsetzung mit einer aktivierten Carbonsäure der Formel Va in den entsprechenden Ether IIg überführt und IIg anschließend zu IIa spaltet. 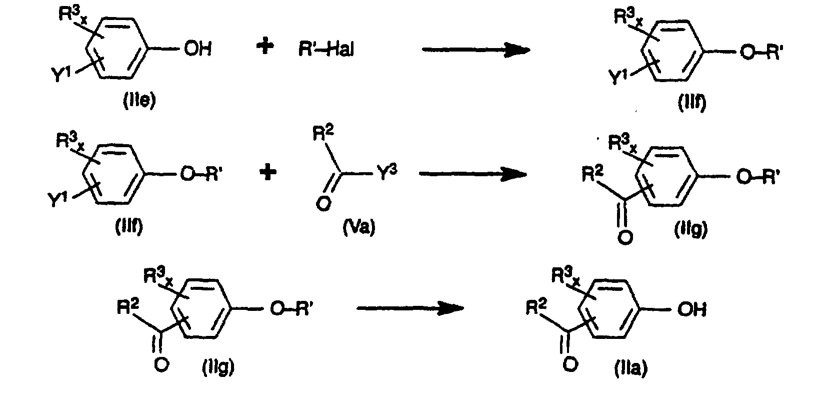
a) Die Veretherung von IIe zu IIf erfolgt üblicherweise bei Temperaturen von 0°C bis 120°C, vorzugsweise 20°C bis 80°C in Gegenwart eines inerten Lösungsmittels.
   Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, sowie Dimethylsulfoxid und Dimethylformamid, besonders bevorzugt Dimethylformamid. Es können auch Gemische der genannten Lösungsmittel verwendet werden. Das Phenol IIe und das Alkylhalogenid werden im allgemeinen in äquimolaren Mengen miteinander umgesetzt. Es kann für die Ausbeute vorteilhaft sein, das Alkylhalogenid in einem Überschuß bezogen auf IIe oder als Lösungsmittel einzusetzen.
b) Die Umsetzung des Ethers IIf mit der aktivierten Carbonsäure Va erfolgt im allgemeinen und im besonderen unter den bei der Herstellung der Verbindungen IIa aus den Verbindungen IIc beschriebenen Bedingungen.
c) Die Etherspaltung von IIg nach IIa erfolgt üblicherweise in einem inerten Lösungsmittel bei Temperaturen von 0°C bis 130°C, vorzugsweise 60°C bis 100°C in Gegenwart einer Säure.
   Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Nitrile wie Acetonitril und Propionitril, Alkohole wie Methanol, Ethanol, n-Propanol, Isopropanol, n-Butanol und tert.-Butanol sowie Dimethylsulfoxid und Dimethylformamid, besonders bevorzugt Methylenchlorid.
   Es können auch Gemische der genannten Lösungsmittel verwendet werden.
   Als Säuren und saure Katalysatoren finden anorganische Säuren wie Fluorwasserstoffsäure, Salzsäure, Bromwasserstoffsäure, Schwefelsäure und Perchlorsäure, Lewis-Säuren wie Bortrifluorid, Aluminiumtrichlorid, Eisen-III-chlorid, Zinn-IV-chlorid, Titan-IV-chlorid und Zink-II-chlorid, sowie organische Säuren wie Ameisensäure, Essigsäure, Propionsäure, Oxalsäure, Zitronensäure und Trifluoressigsäure Verwendung. Die Säuren werden im allgemeinen in katalytischen Mengen eingesetzt, sie können aber auch äquimolar, im Überschuß oder gegebenenfalls als Lösungsmittel verwendet werden.

Die Zwischenprodukte der Formel IVa können außerdem dadurch erhalten werden, daß man einen Ether der Formel IVb entweder
a) in Gegenwart einer metallorganischen Base in einem inerten Lösungsmittel mit einer aktivierten Carbonsäure der Formel Va oder
b) in einem inerten Lösungsmittel mit einer Zinn-organischen Verbindung der Formel VI
umsetzt.

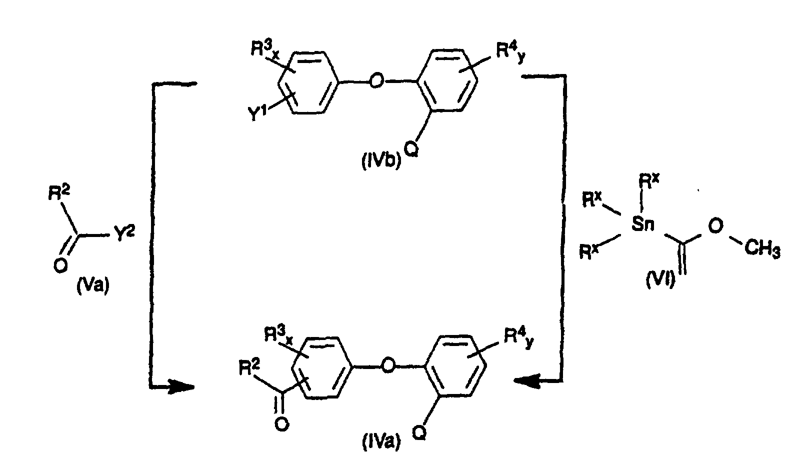

Y¹ in der Formel IVb steht für ein Halogenatom wie Fluor, Chlor, Brom und Iod, insbesondere Brom und Iod.

Y² in der Formel Va steht für ein Halogenatom wie Fluor, Chlor, Brom und Iod, insbesondere Chlor.

Die Reste R^{x} in der Formel VI sind voneinander unabhängig und stehen für Alkyl.
a) Die Umsetzung des Ethers IVb mit der aktivierten Carbonsäure Va erfolgt im allgemeinen und im besonderen unter den bei der Herstellung der Verbindungen IIa aus den Verbindungen IIc beschriebenen Bedingungen.
b) Die Umsetzung des Ethers IVb mit der Zinn-organischen Verbindung VI erfolgt üblicherweise in einem inerten Lösungsmittel bei Temperaturen von -70°C bis 40°C, vorzugsweise -70°C bis 0°C in Gegenwart eines Katalysators wie Pd[P(C₆H₅)₃]₃ und PdCl₂.
   Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, Ether wie Diethylether, Diisopropylether, tert.-Butylmethylether, Dioxan, Anisol und Tetrahydrofuran, Nitrile wie Acetonitril und Propionitril, sowie Dimethylsulfoxid und Dimethylformamid, besonders bevorzugt Tetrahydrofuran und Diethylether. Es können auch Gemische der genannten Lösungsmittel verwendet werden.
   Die Edukte werden im allgemeinen in äquimolaren Mengen miteinander umgesetzt. Es kann für die Ausbeute vorteilhaft sein, die Zinn-organische Verbindung VI in einem Überschuß bezogen auf den Ether IVb einzusetzen.

Verbindungen der Formel I, in denen n für 1 steht, erhält man bevorzugt dadurch, daß man einen Diphenylether der Formel IVc in einem inerten Lösungsmittel in Gegenwart von Salzsäure mit einem Cyanid R²-C≡N in das Hydroxamsäureester-chlorid VIIc überführt und VIIc anschließend mit einem O-substituierten Hydroxylamin (R¹-ONH₂) oder dessen Salz zu I umsetzt.

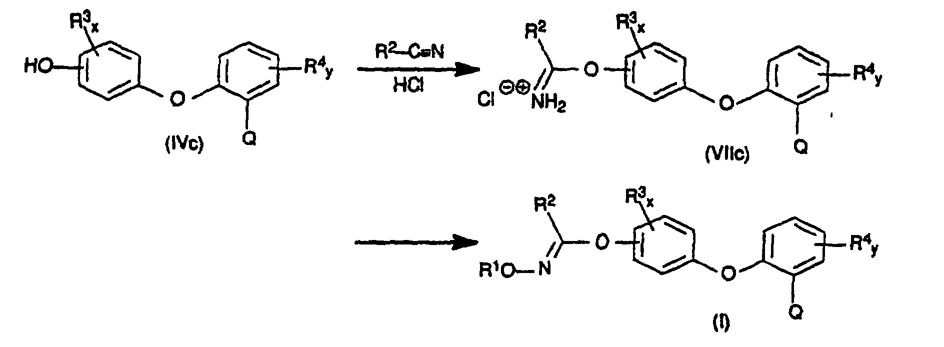

Die Umsetzung zum Hydroxamsäureester-chlorid VIIc erfolgt in allgemeinen und im besonderen nach den in US-A 4,743,701 beschriebenen Bedingungen.

Die Umsetzung von VIIc nach I verläuft im allgemeinen und im besonderen entsprechend den vorstehend für die Umsetzung von IIa zu IIb beschriebenen Bedingungen.

Nach einem besonders bevorzugten Verfahren erhält man die Verbindungen I, in denen n für 0 und Q für C(CO₂CH₃)=NOCH₃ steht, dadurch, daß man ein Phenol der Formel IIa in einem inerten Lösungsmittel mit einem Halogenbenzol der Formel IIIb in den entsprechenden Ether der Formel VIIa überführt, VIIa anschließend mit einem O-substituierten Hydroxylamin (R¹-ONH₂) oder dessen Salz zu VIIb umsetzt und VIIb mit O-Methylhydroxylamin (CH₃-ONH₂) oder dessen Salz umsetzt.

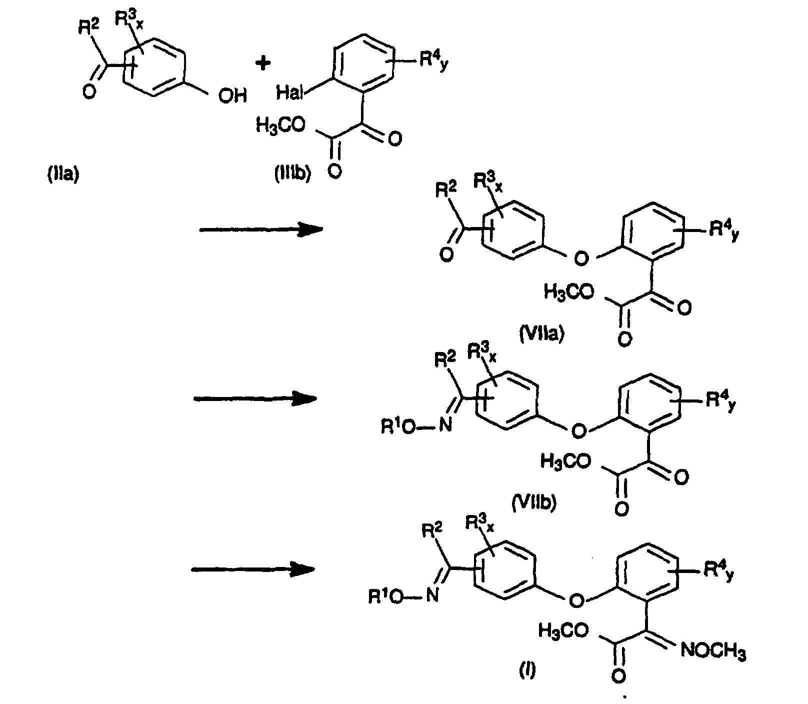

Die Umsetzung von IIa mit IIIb erfolgt gemäß den vorstehend für die Umsetzung von IIb mit IIIa beschriebenen Bedingungen.

Die Umsetzung von VIIa zu VIIb erfolgt gemäß den vorstehend für die Umsetzung von IIa zu IIb beschriebenen Bedingungen.

Die Umsetzung von VIIb zu I erfolgt gemäß den vorstehend für die Umsetzung von IIa zu IIb beschriebenen Bedingungen.

Nach einem besonders bevorzugten Verfahren können die Verbindungen VIIb auch dadurch erhalten werden, daß man eine Verbindung der Formel IIb mit einem Halogenbenzol der Formel IIIb umsetzt.

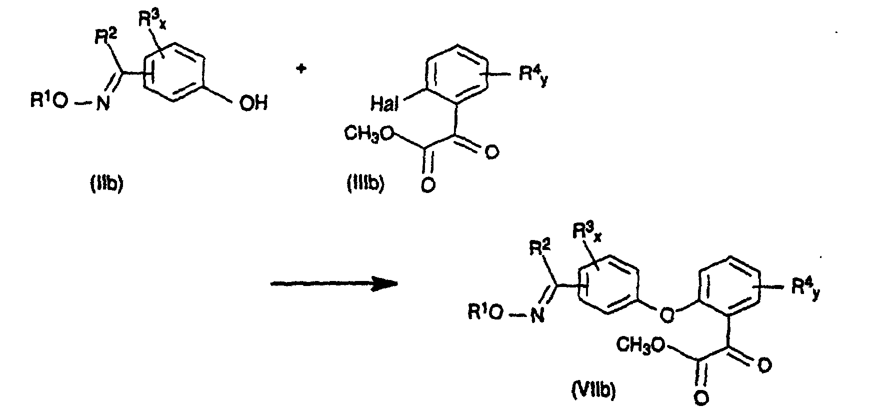

Die Umsetzung erfolgt im allgemeinen und im besonderen gemäß den vorstehend beschriebenen Bedingungen.

In analoger Weise erhält man die Verbindungen der Formel I, in denen n für 0 und Q für C(CONHCH₃)=NOCH₃ steht, besonders bevorzugt dadurch, daß man eine Verbindung der Formel IIb zunächst in einem inerten Lösungsmittel mit einem Halogenbenzol der Formel IIIc in den entsprechenden Ether der Formel VIId überführt und VIId mit O-Methylhydroxylamin (CH₃O-NH₂) oder dessen Salz umsetzt.

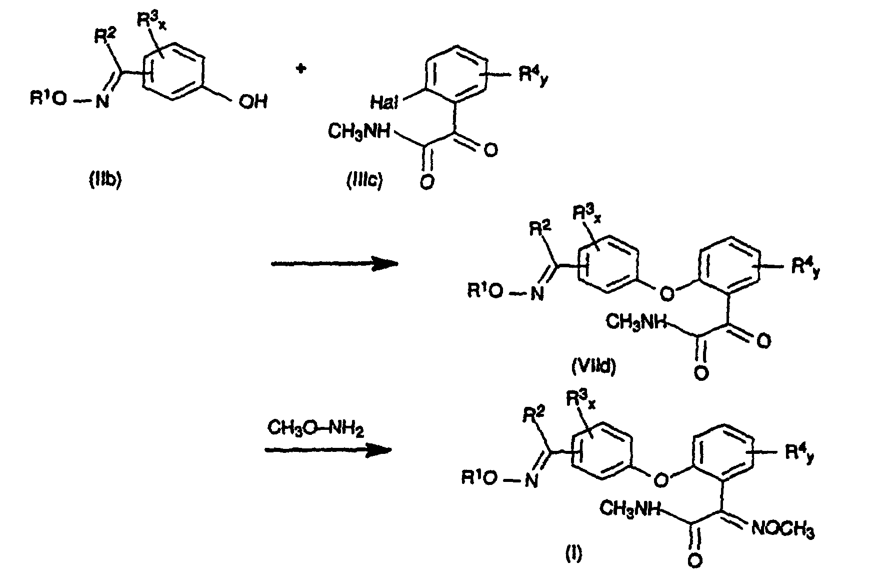

Hal in der Formel IIIc bedeutet ein Halogenatom, insbesondere Fluor.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwi-schen- und Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Besonders geeignet für die Herstellung der Verbindungen I, in denen n für 0 steht, sind Zwischenprodukte der allgemeinen Formel IV 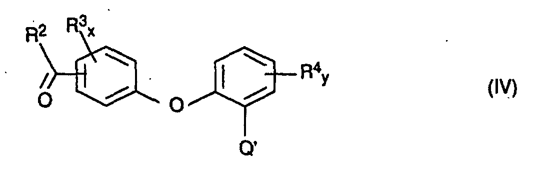 in denen R², R³, R⁴, x und y die vorstehend gegebene Bedeutung haben und Q' für eine der folgenden Gruppen steht:
CH₂-CO₂CH₃, C(O)-CO₂CH₃ oder einen Rest Q.

Außerdem werden für die Herstellung der Verbindungen I Zwischenprodukte der allgemeinen Formel IV' bevorzugt, 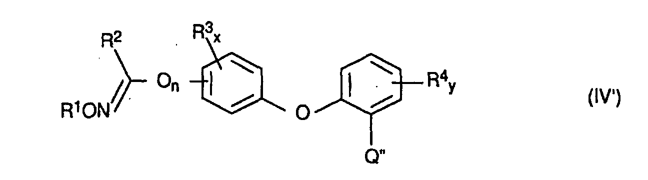 in denen R¹, R², R³, R⁴, n, x und y die vorstehend gegebene Bedeutung haben und Q" für C(O)-CO₂CH₃ steht.

Die Verbindungen I können bei der Herstellung aufgrund ihrer C=C- und C=N-Doppelbindungen als E/Z-Isomerengemische anfallen, die z.B. durch Kristallisation oder Chromatographie in üblicher Weise in die Einzelverbindungen getrennt werden können.

Sofern bei der Synthese Isomerengemische anfallen, ist im allgemeinen jedoch eine Trennung nicht unbedingt erforderlich, da sich die einzelnen Isomere teilweise während der Aufbereitung für die Anwendung oder bei der Anwendung (z.B. unter Licht-, Säure- oder Baseneinwirkung) ineinander umwandeln können. Entsprechende Umwandlungen können auch nach der Anwendung, beispielsweise bei der Behandlung von Pflanzen in der behandelten Pflanze oder im zu bekämpfenden Schadpilz oder tierischen Schädling erfolgen.

In Bezug auf die C=NOR¹-Doppelbindung werden hinsichtlich ihrer Wirksamkeit die E-Isomere der Verbindungen I bevorzugt (Konfiguration bezogen auf die R²-Gruppe im Verhältnis zur OR¹-Gruppe).

Bei den in den vorstehenden Formeln angegebenen Definitionen der Symbole wurden Sammelbegriffe verwendet, die allgemein repräsentativ für die folgenden Substituenten stehen:
**Halogen:** Fluor, Chlor, Brom und Jod;
**Alkyl:** gesättigte, geradkettige oder verzweigte Kohlenwasserstoffreste mit 1 bis 4, 6, 8 oder 10 Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
**Halogenalkyl:** geradkettige oder verzweigte Alkylgruppen mit 1 bis 10 Kohlenstoffatomen (wie vorstehend genannt), wobei in diesen Gruppen teilweise oder vollständig die Wasserstoffatome durch Halogenatome wie vorstehend genannt ersetzt sein können, z.B. C₁-C₂-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
**Alkoxy:** geradkettige oder verzweigte Alkylgruppen mit 1 bis 10 Kohlenstoffatomen (wie vorstehend genannt), welche über ein Sauerstoffatom (-O-) an das Gerüst gebunden sind;
**Alkenyl:** ungesättigte, geradkettige oder verzweigte Kohlenwasserstoffreste mit 2 bis 4, 6, 8 oder 10 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1propenyl und 1-Ethyl-2-methyl-2-propenyl;
**Halogenalkenyl:** ungesättigte, geradkettige oder verzweigte Kohlenwasserstoffreste mit 2 bis 10 Kohlenstoffatomen und einer Doppelbindung in einer beliebigen Position (wie vorstehend genannt), wobei in diesen Gruppen die Wasserstoffatome teilweise oder vollständig gegen Halogenatome wie vorstehend genannt, insbesondere Fluor, Chlor und Brom, ersetzt sein können;
**Alkenyloxy:** ungesättigte, geradkettige oder verzweigte Kohlenwasserstoffreste mit 3 bis 10 Kohlenstoffatomen und einer Doppelbindung in einer beliebigen, nicht zum Heteroatom benachbarten, Position (wie vorstehend genannt), welche über ein Sauerstoffatom (-O-) an das Gerüst gebunden sind;
**Alkinyl:** geradkettige oder verzweigte Kohlenwasserstoffgruppen mit 2 bis 4, 6, 8 oder 10 Kohlenstoffatomen und einer Dreifachbindung in einer beliebigen Position, z.B. C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;
**Halogenalkinyl:** ungesättigte, geradkettige oder verzweigte Kohlenwasserstoffreste mit 2 bis 10 Kohlenstoffatomen und einer Dreifachbindung in einer beliebigen Position (wie vorstehend genannt), wobei in diesen Gruppen die Wasserstoffatome teilweise oder vollständig gegen Halogenatome wie vorstehend genannt, insbesondere Fluor, Chlor und Brom, ersetzt sein können;
**Alkinyloxy**: ungesättigte, geradkettige oder verzweigte Kohlenwasserstoffreste mit 3 bis 10 Kohlenstoffatomen und einer Dreifachbindung in einer beliebigen, nicht zum Heteroatom benachbarten, Position (wie vorstehend genannt), welche über ein Sauerstoffatom (-O-) an das Gerüst gebunden sind;
**Halogenalkinyloxy:** ungesättigte, geradkettige oder verzweigte Alkinyloxygruppen mit 3 bis 10 Kohlenstoffatomen (wie vorstehend genannt), wobei in diesen Gruppen die Wasserstoffatome teilweise oder vollständig gegen Halogenatome wie vorstehend genannt, insbesondere Fluor, Chlor und Brom, ersetzt sein können;
**Cycloalkyl:** monocyclische, gesättigte Kohlenwasserstoffgruppen mit 3 bis 6, 8, 10 oder 12 Kohlenstoffringgliedern, z.B. C₃-C₈-Cycloalkyl wie Cyclopropyl, Cyclobutyl, Cyclopentyl, Cyclohexyl, Cycloheptyl und Cyclooctyl;
**Cycloalkoxy:** monocyclische, gesättigte Kohlenwasserstoffgruppen mit 3 bis 12 Kohlenstoffringgliedern (wie vorstehend genannt), welche über ein Sauerstoffatom (-O-) an das Gerüst gebunden sind;
**gesättigtes oder partiell ungesättigter cyclischer Rest, welcher neben Kohlenstoffatomen als Ringglieder Heteroatome aus der Gruppe Sauerstoff, Schwefel oder Stickstoff enthalten kann:** Cycloalkyl mit 3 bis 12 Kohlenstoffringgliedern wie vorstehend genannt oder 5- oder 6-gliedrige Heterocyclen (Heterocyclyl) enthaltend neben Kohlenstoffringgliedern ein bis drei Stickstoffatome und/oder ein Sauerstoff- oder Schwefelatom oder ein oder zwei Sauerstoff- und/oder Schwefelatome, z.B. 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,4-Dihydrofur-3-yl, 2,3-Dihydrothien-2-yl, 2,3-Dihydrothien-3-yl, 2,4-Dihydrothien-2-yl, 2,4-Dihydrothien-3-yl, 2,3-Pyrrolin-2-yl, 2,3-Pyrrolin-3-yl, 2,4-Pyrrolin-2-yl, 2,4-Pyrrolin-3-yl, 2,3-Isoxazolin-3-yl, 3,4-Isoxazolin-3-yl, 4,5-Isoxazolin-3-yl, 2,3-Isoxazolin-4-yl, 3,4-Isoxazolin-4-yl, 4,5-Isoxazolin-4-yl, 2,3-Isoxazolin-5-yl, 3,4-Isoxazolin-5-yl, 4,5-Isoxazolin-5-yl, 2,3-Isothiazolin-3-yl, 3,4-Isothiazolin-3-yl, 4,5-Isothiazolin-3-yl, 2,3-Isothiazolin-4-yl, 3,4-Isothiazolin-4-yl, 4,5-Isothiazolin-4-yl, 2,3-Isothiazolin-5-yl, 3,4-Isothiazolin-5-yl, 4,5-Isothiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-1-yl, 3,4-Dihydropyrazol-3-yl, 3,4-Dihydropyrazol-4-yl, 3,4-Dihydropyrazol-5-yl, 4,5-Dihydropyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 3,4-Dihydrooxazol-2-yl, 3,4-Dihydrooxazol-3-yl, 3,4-Dihydrooxazol-4-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Tetrahydropyridazinyl, 4-Tetrahydropyridazinyl, 2-Tetrahydropyrimidinyl, 4-Tetrahydropyrimidinyl, 5-Tetrahydropyrimidinyl, 2-Tetrahydropyrazinyl, 1,3,5-Tetrahydro-triazin-2-yl und 1,2,4-Tetrahydrotriazin-3-yl;
**Heterocyclyloxy:** ein 5- oder 6-gliedriger Heterocyclus (wie vorstehend genannt), welcher über ein Sauerstoffatom (-O-) an das Gerüst gebunden ist;
**Aryl:** ein ein- bis dreikerniges aromatisches Ringsystem enthaltend 6 bis 14 Kohlenstoffringglieder, z.B. Phenyl, Naphthyl und Anthracenyl;
**Aryloxy:** ein ein- bis dreikerniges aromatisches Ringsystem (wie vorstehend genannt), welches über ein Sauerstoffatom (-O-) an das Gerüst gebunden ist;
**aromatisches Ringsystem, welches neben Kohlenstoffringgliedern Heteroatome aus der Gruppe Sauerstoff, Schwefel und Stickstoff enthalten kann:** Aryl wie vorstehend genannt oder ein- oder zweikerniges Heteroaryl, z.B.
   - 5-gliedriges Heteroaryl, enthaltend ein bis vier Stickstoffatome oder ein bis drei Stickstoffatome und ein Schwefel- oder Sauerstoffatom: 5-Ring Heteroarylgruppen, welche neben Kohlenstoffatomen ein bis vier Stickstoffatome oder ein bis drei Stickstoffatome und ein Schwefel- oder Sauerstoffatom als Ringglieder enthalten können, z.B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl;
   - benzokondensiertes 5-gliedriges Heteroaryl, enthaltend ein bis drei Stickstoffatome oder ein Stickstoffatom und ein Sauerstoff- oder Schwefelatom: 5-Ring Heteroarylgruppen, welche neben Kohlenstoffatomen ein bis vier Stickstoffatome oder ein bis drei Stickstoffatome und ein Schwefel- oder Sauerstoffatom als Ringglieder enthalten können, und in welchen zwei benachbarte Kohlenstoffringglieder oder ein Stickstoff- und ein benachbartes Kohlenstoffringglied durch eine Buta-1,3-dien-1,4-diylgruppe verbrückt sein können;
   - über Stickstoff gebundenes 5-gliedriges Heteroaryl, enthaltend ein bis vier Stickstoffatome, oder über Stickstoff gebundenes benzokondensiertes 5-gliedriges Heteroaryl, enthaltend ein bis drei Stickstoffatome: 5-Ring Heteroarylgruppen, welche neben Kohlenstoffatomen ein bis vier Stickstoffatome bzw. ein bis drei Stickstoffatome als Ringglieder enthalten können, und in welchen zwei benachbarte Kohlenstoffringglieder oder ein Stickstoff- und ein benachbartes Kohlenstoffringglied durch eine Buta-1,3-dien-1,4-diylgruppe verbrückt sein können, wobei diese Ringe über eines der Stickstoffringglieder an das Gerüst gebunden sind;
   - 6-gliedriges Heteroaryl, enthaltend ein bis drei bzw. ein bis vier Stickstoffatome: 6-Ring Heteroarylgruppen, welche neben Kohlenstoffatomen ein bis drei bzw. ein bis vier Stickstoffatome als Ringglieder enthalten können, z.B. 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl;
**Alkylen:** divalente unverzweigte Ketten aus 3 bis 5 CH₂-Gruppen, z.B. CH₂, CH₂CH₂, CH₂CH₂CH₂, CH₂CH₂CH₂CH₂ und CH₂CH₂CH₂CH₂CH₂;
**Oxyalkylen:** divalente unverzweigte Ketten aus 2 bis 4 CH₂-Gruppen, wobei eine Valenz über ein Sauerstoffatom an das Gerüst gebunden ist, z.B. OCH₂CH₂, OCH₂CH₂CH₂ und OCH₂CH₂CH₂CH₂;
**Oxyalkylenoxy:** divalente unverzweigte Ketten aus 1 bis 3 CH₂-Gruppen, wobei beide Valenzen über ein Sauerstoffatom an das Gerüst gebunden ist, z.B. OCH₂O, OCH₂CH₂O und OCH₂CH₂CH₂O;
**organischer Rest:** ggf. subst. Alkyl, Alkenyl, Alkinyl, Cycloalkyl, Heterocyclyl, Aryl oder Hetaryl.

Der Zusatz *"ggf. subst."* in Bezug auf *Alkyl-, Alkenyl- und Alkinylgruppen* soll zum Ausdruck bringen, daß diese Gruppen partiell oder vollständig halogeniert sein können *[d.h. die Wasserstoffatome dieser Gruppen können teilweise oder vollständig durch gleiche oder verschiedene Halogenatome wie vorstehend genannt (vorzugsweise Fluor, Chlor oder Brom) ersetzt sein]* und/oder einen bis drei (vorzugsweise einen) der folgenden Reste tragen können:
- Cyano, Nitro, Hydroxy, Amino, Formyl, Carboxyl, Aminocarbonyl, Alkoxy, Halogenalkoxy, Alkylthio, Halogenalkylthio, Alkylamino, Dialkylamino, Alkylcarbonyl, Alkoxycarbonyl, Alkylcarbonyloxy, Alkylaminocarbonyl, Dialkylaminocarbonyl, Alkylcarbonylamino, Alkoxycarbonylamino, Alkylcarbonyl-N-alkylamino und Alkylcarbonyl-N-alkylamino, wobei die Alkylgruppen in diesen Resten vorzugsweise 1 bis 6 Kohlenstoffatome, insbesondere 1 bis 4 Kohlenstoffatome enthalten;
- unsubstituiertes oder durch übliche Gruppen substituiertes Cycloalkyl, Cycloalkoxy, Cycloalkylthio, Cycloalkylamino, Cycloalkyl-N-alkylamino, Heterocyclyl, Heterocyclyloxy, Heterocyclylthio, Heterocyclylamino oder Heterocyclyl-N-alkylamino, wobei die cyclischen Systeme 3 bis 12 Ringglieder, vorzugsweise 2 bis 8 Ringglieder, insbesondere 3 bis 6 Ringglieder enthalten und die Alkylgruppen in diesen Resten vorzugsweise 1 bis 6 Kohlenstoffatome, insbesondere 1 bis 4 Kohlenstoffatome enthalten;
- unsubstituiertes oder durch übliche Gruppen substituiertes Aryl, Aryloxy, Arylthio, Arylamino, Aryl-N-alkylamino, Arylalkoxy, Arylalkylthio, Arylalkylamino, Arylalkyl-N-alkylamino, Hetaryl, Hetaryloxy, Hetarylthio, Hetarylamino, Hetaryl-N-alkylamino, Hetarylalkoxy, Hetarylalkylthio, Hetarylalkylamino und Hetarylalkyl-N-alkylamino, wobei die Arylreste vorzugsweise 6 bis 10 Ringglieder, insbesondere 6 Ringglieder (Phenyl) enthalten, die Hetarylreste insbesondere 5 oder 6 Ringglieder enthalten und die Alkylgruppen in diesen Resten vorzugsweise 1 bis 6 Kohlenstoffatome, insbesondere 1 bis 4 Kohlenstoffatome enthalten.

Der Zusatz *"ggf. subst"* in Bezug auf die *cyclischen (gesättigten, ungesättigten oder aromatischen) Gruppen* soll zum Ausdruck bringen, daß diese Gruppen partiell oder vollständig halogeniert sein können *[d.h. die Wasserstoffatome dieser Gruppen können teilweise oder vollständig durch gleiche oder verschiedene Halogenatome wie vorstehend genannt (vorzugsweise Fluor, Chlor oder Brom, insbesondere Fluor oder Chlor) ersetzt sein]* und/oder einen bis vier (insbesondere einen bis drei) der folgenden Reste tragen können:
- Cyano, Nitro, Hydroxy, Amino, Carboxyl, Aminocarbonyl, Alkyl, Haloalkyl, Alkenyl, Haloalkenyl, Alkenyloxy, Haloalkenyloxy, Alkinyl, Haloalkinyl, Alkinyloxy, Haloalkinyloxy, Alkoxy, Halogenalkoxy, Alkylthio, Halogenalkylthio, Alkylamino, Dialkylamino, Alkylcarbonyl, Alkoxycarbonyl, Alkylcarbonyloxy, Alkylaminocarbonyl, Dialkylaminocarbonyl, Alkylcarbonylamino, Alkoxycarbonylamino, Alkylcarbonyl-N-alkylamino und Alkylcarbonyl-N-alkylamino, wobei die Alkylgruppen in diesen Resten vorzugsweise 1 bis 6 Kohlenstoffatome, insbesondere 1 bis 4 Kohlenstoffatome enthalten und die genannten Alkenyl- oder Alkinylgruppen in diesen Resten 2 bis 8, vorzugsweise 2 bis 6, insbesondere 2 bis 4 Kohlenstoffatome enthalten;
und/oder einen bis drei (insbesondere einen) der folgenden Reste:
- unsubstituiertes oder durch übliche Gruppen substituiertes Cycloalkyl, Cycloalkoxy, Cycloalkylthio, Cycloalkylamino, Cycloalkyl-N-alkylamino, Heterocyclyl, Heterocyclyloxy, Heterocyclylthio, Heterocyclylamino oder Heterocyclyl-N-alkylamino, wobei die cyclischen Systeme 3 bis 12 Ringglieder, vorzugsweise 2 bis 8 Ringglieder, insbesondere 3 bis 6 Ringglieder enthalten und die Alkylgruppen in diesen Resten vorzugsweise 1 bis 6 Kohlenstoffatome, insbesondere 1 bis 4 Kohlenstoffatome enthalten;
- unsubstituiertes oder durch übliche Gruppen substituiertes Aryl, Aryloxy, Arylthio, Arylamino, Aryl-N-alkylamino, Arylalkoxy, Arylalkylthio, Arylalkylamino, Arylalkyl-N-alkylamino, Hetaryl, Hetaryloxy, Hetarylthio, Hetarylamino, Hetaryl-N-alkylamino, Hetarylalkoxy, Hetarylalkylthio, Hetarylalkylamino und Hetarylalkyl-N-alkylamino, wobei die Arylreste vorzugsweise 6 bis 10 Ringglieder, insbesondere 6 Ringglieder (Phenyl) enthalten, die Hetarylreste insbesondere 5 oder 6 Ringglieder enthalten und die Alkylgruppen in diesen Resten vorzugsweise 1 bis 6 Kohlenstoffatome, insbesondere 1 bis 4 Kohlenstoffatome enthalten;
und/oder einen oder zwei (insbesondere einen) der folgenden Reste:
- Formyl,
- CRⁱⁱⁱ=NOR^{iv} [wobei Rⁱⁱⁱ Wasserstoff, Alkyl, Cycloalkyl und Aryl und R^{iv} Alkyl, Alkenyl, Halogenalkenyl, Alkinyl und Arylalkyl bedeutet (wobei die genannten Alkylgruppen vorzugsweise 1 bis 6 Kohlenstoffatome, insbesondere 1 bis 4 Kohlenstoffatome, enthalten, die genannten Cycloalkylgruppen, Alkenylgruppen und Alkinylgruppen vorzugsweise 3 bis 8, insbesondere 3 bis 6, Kohlenstoffatome enthalten) und Aryl insbesondere Phenyl bedeutet, welches unsubstituiert ist oder durch übliche Gruppen substituiert sein kann] oder
- NR^{v}-CO-D-R^{vi} [wobei R^{v} für Wasserstoff, Hydroxy, C₁-C₆-Alkyl, C₂-C₆-Alkenyl, C₂-C₆-Alkinyl, C₁-C₆-Alkoxy, C₂-C₆-Alkenyloxy, C₂-C₆-Alkinyloxy, C₁-C₆-Alkoxy-C₁-C₆-alkyl, C₁-C₆-Alkoxy-C₁-C₆-alkoxy und C₁-C₆-Alkoxycarbonyl steht, R^{vi} für Wasserstoff, C₁-C₆-Alkyl, C₂-C₆-Alkenyl, C₂-C₆-Alkinyl, C₃-C₆-Cycloalkyl, C₃-C₆-Cycloalkenyl, Aryl, Aryl-C₁-C₆-alkyl, Hetaryl und Hetaryl-C₁-C₆-alkyl steht und D eine direkte Bindung, Sauerstoff oder Stickstoff bedeutet, wobei der Stickstoff eine der bei R^{vi} genannten Gruppen tragen kann],
und/oder bei denen zwei benachbarte C-Atome der cyclischen Systeme eine C₃-C₅-Alkylen-, C₃-C₅-Alkenylen-, Oxy-C₂-C₄alkylen-, Oxy-C₁-C₃-alkylenoxy, Oxy-C₂-C₄-alkenylen-, Oxy-C₂-C₄-alkenylenoxy- oder Butadiendiylgruppe tragen können, wobei diese Brücken ihrerseits partiell oder vollständig halogeniert sein können und/oder einen bis drei, insbesondere einen oder zwei der folgenden Reste tragen können:
- C₁-C₄-Alkyl, C₁-C₄-Halogenalkyl, C₁-C₄-Alkoxy, C₁-C₄-Halogenalkoxy und C₁-C₄-Alkylthio.

Unter üblichen Gruppen sind insbesondere die folgenden Substituenten zu verstehen: Halogen, Cyano, Nitro, C₁-C₄-Alkyl, C₁-C₄-Halogenalkyl, C₁-C₄-Alkoxy, C₁-C₄-Halogenalkoxy, C₁-C₄-Alkylamino, Di-C₁-C₄-Alkylamino und C₁-C₄-Alkylthio.

Besonders bevorzugt sind Verbindungen der Formel I, in denen R¹ für Wasserstoff oder eine der folgenden Gruppen steht: ggf. subst. Alkyl, Alkenyl, Alkinyl, Cycloalkyl, Heterocyclyl, Aryl oder Heteroaryl.

Insbesondere werden Verbindungen I bevorzugt, in denen R¹ für Wasserstoff steht.

Außerdem werden Verbindungen I besonders bevorzugt, in denen R¹ für ggf. subst. C₁-C₆-Alkyl steht.

Gleichermaßen besonders bevorzugt sind Verbindungen I, in denen R¹ für ggf. subst. C₃-C₆-Alkenyl steht.

Daneben werden Verbindungen I besonders bevorzugt, in denen R¹ für ggf. subst. C₃-C₆-Alkinyl steht.

Insbesondere werden auch Verbindungen I bevorzugt, in denen R¹ für C₁-C₆-Halogenalkyl steht.

Außerdem werden Verbindungen I besonders bevorzugt, in denen R¹ für C₃-C₆-Halogenalkenyl steht.

Gleichermaßen besonders bevorzugt sind Verbindungen I, in denen R¹ für Aryl-C₁-C₂-alkyl steht, wobei der Arylrest gegebenfalls substituiert sein kann.

Besonders bevorzugt sind Verbindungen I, in denen R¹ für Aryl-C₁-C₂-alkyl steht, wobei der Arylrest partiell oder vollständig halogeniert sien kann und/oder eine bis drei der folgenden Gruppen tragen kann: C₁-C₄-Alkyl, C₁-C₄-Halogenalkyl und C₁-C₄-Alkoxy.

Daneben werden Verbindungen I besonders bevorzugt, in denen R¹ für C₃-C₆-Cycloalkyl-C₁-C₂-alkyl steht, wobei der Cycloalkylrest gegebenfalls substituiert sein kann.

Insbesondere werden Verbindungen I bevorzugt, in denen R¹ für C₃-C₆-Cycloalkyl-C₁-C₂-alkyl steht, wobei der Cycloalkylrest partiell oder vollständig halogeniert sein kann und/oder eine bis drei C₁-C₄-Alkylgruppen tragen kann.

Desweiteren werden Verbindungen I besonders bevorzugt, in denen R¹ für Hetaryl-C₁-C₂-alkyl steht, wobei der Hetarylrest gegebenfalls substituiert sein kann.

Insbesondere werden Verbindungen I bevorzugt, in denen R¹ für Hetaryl-C₁-C₂-alkyl steht, wobei der Hetarylrest partiell oder vollständig halogeniert sein kann und/oder eine bis drei der folgenden Gruppen tragen kann: C₁-C₄-Alkyl, C₁-C₄-Halogenalkyl und C₁-C₄-Alkoxy.

Außerdem werden Verbindungen der Formel I besonders bevorzugt, in denen R² für Wasserstoff oder eine der folgenden Gruppen steht: ggf. subst. Alkyl, Alkenyl, Alkinyl, Cycloalkyl, Heterocyclyl, Aryl oder Heteroaryl, wobei diese Gruppen direkt (über ein Kohlenstoff-Atom) oder über ein Sauerstoff-, Schwefel- oder Stickstoff-Atom an das Gerüst gebunden sind.

Insbesondere werden Verbindungen I bevorzugt, in denen R² für ggf. subst. C₁-C₆-Alkyl steht.

Außerdem werden Verbindungen I besonders bevorzugt, in denen R² für ggf. subst. C₂-C₆-Alkenyl steht.

Gleichermaßen besonders bevorzugt sind Verbindungen I, in denen R² für ggf. subst. C₂-C₆-Alkinyl steht.

Daneben werden Verbindungen I besonders bevorzugt, in denen R² für C₃-C₆-Cycloalkyl steht.

Insbesondere werden auch Verbindungen I bevorzugt, in denen R² für Aryl steht.

Außerdem werden Verbindungen I besonders bevorzugt, in denen R² für Hetaryl steht.

Desweiteren werden Verbindungen I besonders bevorzugt, in denen x für 0, 1 oder 2 steht, wobei die Reste R³ verschieden sein können, wenn x für 2 steht.

Insbesondere werden Verbindungen I bevorzugt, in denen x für 0 oder 1 steht.

Außerdem werden Verbindungen der Formel I besonders bevorzugt, in denen R³ für Cyano, Nitro, Halogen oder eine der folgenden Gruppen steht: ggf. subst. Alkyl, Alkenyl, Alkinyl, Cycloalkyl, Heterocyclyl, Aryl oder Heteroaryl, wobei diese Gruppen direkt (über ein Kohlenstoff-Atom) oder über ein Sauerstoff-, Schwefel- oder Stickstoff-Atom an das Gerüst gebunden sind.

Insbesondere werden Verbindungen I bevorzugt, in denen R³ für Halogen, insbesondere Fluor, Chlor und Brom, steht.

Außerdem werden Verbindungen I besonders bevorzugt, in denen R³ für C₁-C₂-Alkyl steht.

Gleichermaßen besonders bevorzugt sind Verbindungen I, in denen R³ für C₁-C₂-Alkoxy steht.

Daneben werden Verbindungen I besonders bevorzugt, in denen R³ für Cyano steht.

Insbesondere werden auch Verbindungen I bevorzugt, in denen R³ für Nitro steht.

Desweiteren werden Verbindungen I besonders bevorzugt, in denen y für 0, 1, 2 oder 3 steht, wobei die Reste R⁴ verschieden sein können, wenn y für 2 oder 3 steht.

Insbesondere werden Verbindungen I bevorzugt, in denen y für 0 oder 1 steht.

Außerdem werden Verbindungen I besonders bevorzugt, in denen R⁴ Cyano, Halogen, C₁-C₃-Alkyl, C₁-C₂-Halogenalkyl oder C₁-C₃-Alkoxy, insbesondere Cyano, Fluor, Chlor, Methyl, Ethyl, Isopropyl, Trifluormethyl, Methoxy oder Ethoxy, bedeutet.

Insbesondere werden Verbindungen I bevorzugt, in denen R⁴ für Methyl steht.

Außerdem werden Verbindungen I besonders bevorzugt, in denen R⁴ für Methoxy steht.

Gleichermaßen besonders bevorzugt sind Verbindungen I, in denen R⁴ für Fluor steht.

Daneben werden Verbindungen I besonders bevorzugt, in denen R⁴ für Chlor steht.

Insbesondere werden auch Verbindungen I bevorzugt, in denen R⁴ für Trifluormethyl steht.

Außerdem werden Verbindungen I besonders bevorzugt, in denen R⁴ für OCH₂O steht.

Die Verbindungen I eignen sich als Fungizide.

Die Verbindungen I zeichnen sich durch eine hervorragende Wirksamkeit gegen ein breites Spektrum von pflanzenpathogenen Pilzen, insbesondere aus der Klasse der Ascomyceten, Phycomyceten und Basidiomyceten, aus. Sie sind zum Teil systemisch wirksam und können im Pflanzenschutz als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zukkerrohr, Wein, Obst- und Zierpflanzen und Gemüsepflanzen wie Gurken, Bohnen und Kürbisgewächsen, sowie an den Samen dieser Pflanzen.

Speziell eignen sie sich zur Bekämpfung folgender Pflanzenkrankheiten: Erysiphe graminis (echter Mehltau) in Getreide, Erysiphe cichoracearum und Sphaerotheca fuliginea an Kürbisgewächsen, Podosphaera leucotricha an Äpfeln, Uncinula necator an Reben, Puccinia-Arten an Getreide, Rhizoctonia-Arten an Baumwolle, Reis und Rasen, Ustilago-Arten an Getreide und Zuckerrohr, Venturia inaequalis (Schorf) an Äpfeln, Helminthosporium-Arten an Getreide, Septoria nodorum an Weizen, Botrytis cinerea (Grauschimmel) an Erdbeeren, Gemüse und Zierpflanzen, Reben, Cercospora arachidicola an Erdnüssen, Pseudocercosporella herpotrichoides an Weizen, Gerste, Pyricularia oryzae an Reis, Phytophthora infestans an Kartoffeln und Tomaten, Fusarium- und Verticillium-Arten an verschiedenen Pflanzen, Plasmopara viticola an Reben, Alternaria-Arten an Gemüse und Obst.

Die Verbindungen I eignen sich außerdem zur Bekämpfung von Schadpilzen im Materialschutz (z.B. Holz, Papier, Fasern bzw. Gewebe) und im Vorratsschutz.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung erfolgt vor oder nach der Infektion der Materialien, Pflanzen oder *Samen durch die Pilze.

Sie können in die üblichen Formulierungen überführt werden, wie Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der erfindungsgemäßen Verbindung gewährleisten. Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B.Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Die fungiziden Mittel enthalten im allgemeinen zwischen 0,1 und 95, vorzugsweise zwischen 0,5 und 90 Gew.-% Wirkstoff.

Die Aufwandmengen liegen bei der Anwendung im Pflanzenschutz je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.

Bei der Anwendung im Material- bzw. Vorratsschutz richtet sich die Aufwandmenge an Wirkstoff nach der Art des Einsatzgebietes und des gewünschten Effekts. Übliche Aufwandmengen sind im Materialschutz beispielsweise 0,001 g bis 2 kg, vorzugsweise 0,005 g bis 1 kg Wirkstoff pro Qubikmeter behandelten Materials.

Die erfindungsgemäßen Mittel können in der Anwendungsform als Fungizide auch zusammen mit anderen Wirkstoffen vorliegen, z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Die folgende Liste von Fungiziden, mit denen die erfindungs-gemäßen Verbindungen gemeinsam angewendet werden können, soll die Kombinationsmöglichkeiten erläutern, nicht aber einschränken:
Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zink-ethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylenbis-dithiocarbamat), N,N'-Polypropylen-bis-(thiocarbamoyl)disulfid;
Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthalsäure-diisopropylester;
heterocyclische Substanzen, wie 2-Heptadecyl-2-imidazolin-acetat, 2,4-Dichlor-6-(o-chloranilino)-s-triazin, O,O-Diethylphthalimidophosphonothioat, 5-Amino-1-[bis-(dimethylamino)-phosphinyl]-3-phenyl-1,2,4- triazol, 2,3-Dicyano-1,4-dithioanthrachinon, 2-Thio-1,3-dithiolo[4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthio-phthalimid,
N-Dichlorfluormethylthio-N',N'-dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Rupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäure-anilid, N-Formyl-N-morpholin-2,2,2-trichlorethylacetal, Piperazin-1,4-diylbis-N-(2,2,2-trichlor-ethyl)-formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclododecyl-morpholin bzw. dessen Salze, N-[3-(p-tert.-Butylphenyl)-2-methylpropyl]-cis-2,6-dimethyl-morpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol, 1-[2-(2,4-Dichlorphenyl)-4-n-propyl-1,3-dioxolan-2-yl-ethyl]-1H-1,2,4-triazol, N-(n-Propyl)-N-(2,4,6-trichlorphenoxyethyl)-N'-imidazol-yl-harnstoff, 1-(4-Chlorphenoxy)-3,3-dimethyl-1-(1H-1,2,4-triazol-1-yl)-2-butanon, 1-(4-Chlorphenoxy)-3,3-dimethyl-1-(1H-1,2,4-triazol-1-yl)-2-butanol, a-(2-Chlorphenyl)-α-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol,
sowie verschiedene Fungizide, wie Dodecylguanidinacetat, 3-[3-(3,5-Dimethyl-2-oxycyclohexyl)-2-hydroxyethyl] -glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alaninmethyl- ester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-[3,5-Dichlorphenyl-5-methyl-5-methoxymethyl]-1,3-oxazolidin- 2, 4-dion, 3- (3,5-Dichlorphenyl)-1-isopropylcarbamoylhydantoin, N- (3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol,
Strobilurine wie Methyl-E-methoxyimino-[α-(o-tolyloxy)-o-tolyl]-acetat, Methyl-E-2-{2-[6-(2-cyanophenoxy)-pyrimidin-4-yloxy]-phenyl}-3-methoxyacrylat, Methyl-E-methoxyimino-[α-(2-phenoxyphenyl)]-acetamid, Methyl-E-methoxyimino-[α-(2,5-dimethyloxy)-o-tolyl]-acetamid,
Anilinopyrimidine wie N-(4,6-Dimethylpyrimidin-2-yl)-anilin, N-[4-Methyl-6-(1-propinyl)-pyrimidin-2-yl]-anilin, N-(4-Methyl-6-cyclopropyl-pyrimidin-2-yl)-anilin,
Phenylpyrrole wie 4-(2,2-difluor-1,3-benzodioxol-4-yl)-pyrrol-3-carbonitril,
Zimtsäureamide wie 3-(4-Chlorphenyl)-3-(3,4-dimethoxyphenyl)-acrylsäuremorpholid.

Die Verbindungen der Formel I sind außerdem geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grandiosella, Earias insulana, Elasmopalpus lignosellus, Eupoecilia ambiguella, Evetria bouliana, Feltia subterranea, Galleria mellonella, Grapholitha funebrana, Grapholitha molesta, Heliothis armigera, Heliothis virescens, Heliothis zea, Hellula undalis, Hibernia defoliaria, Hyphantria cunea, Hyponomeuta malinellus, Keiferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flammea, Pectinophora gossypiella, Peridroma saucia, Phalera bucephala, Phthorimaea operculella, Phyllocnistis citrella, Pieris brassicae, Plathypena scabra, Plutella xylostella, Pseudoplusia includens, Rhyacionia frustrana, Scrobipalpula absoluta, Sitotroga cerealella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrhynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Ortiorrhynchus sulcatus, Otiorrhynchus ovatus, Phaedon cochleariae, Phyllotreta chrysocephala, 5 Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossina morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus lineolaris, *Lygus* pratensis, Nezara viridula, Piesma quadrata, Solubea insularis, Thyanta perditor.

Aus der Ordnung der Pflanzensauger (Homoptera) beispielsweise Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dysaphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schi-5 zoneura lanuginosa, Trialeurodes vaporariorum, Viteus vitifolii.

Aus der Ordnung der Termiten (Isoptera) beispielsweise Calotermes flavicollis, Leucotermes flavipes, Reticulitermes lucifugus, Termes natalensis.

Aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus, Tachycines asynamorus.

Aus der Klasse der Arachnoidea beispielsweise Spinnentiere (Acarina) wie Amblyomma americanum, Amblyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobius megnini, Paratetranychus pilosus, Dermanyssus gallinae, Phyllocoptruta oleivora, Polyphagotarsonemus latus, Psoroptes ovis, Rhipicephalus appendiculatus, Rhipicephalus evertsi, Sarcoptes scabiei, Tetranychus cinnabarinus, Tetranychus kanzawai, Tetranychus pacificus, Tetranychus telarius, Tetranychus urticae.

Aus der Klasse der Nematoden beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycines, Heterodera schachtii, Heterodera trifolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus, Pratylenchus goodeyi.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie soll-5 ten in jedem Fall möglichst die feinste Verteilung der erfindungsgemäßen Wirkstoffe gewährleisten.

Die Wirkstoffkonzentrationen in den anwendungsfertigen Zubereitungen können in größeren Bereichen variiert werden.

Im allgemeinen liegen sie zwischen 0,0001 und 10%, vorzugsweise zwischen 0,01 und 1%.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Die Aufwandmenge an Wirkstoff zur Bekämpfung von Schädlingen beträgt unter Freilandbedingungen 0,1 bis 2,0, vorzugsweise 0,2 bis 1,0 kg/ha.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und 5 Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäure mit Phenol und Formaldehyd, Polyoxyethylenoctylphenylether, ethoxyliertes Isooctylphenol, Octylphenol, Nonylphenol, Alkylphenylpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Die Formulierungen enthalten im allgemeinen zwischen 0,01 und 95Gew.-%, vorzugsweise zwischen 0,1 und 90 Gew.-% des Wirkstoffs. Die Wirkstoffe werden dabei in einer Reinheit von 90% bis 100%, vorzugsweise 95% bis 100% (nach NMR-Spektrum) eingesetzt.

Beispiele für Formulierungen sind:
I. 5 Gew.-Teile einer erfindungsgemäßen Verbindung werden mit 95 Gew.-Teilen feinteiligem Kaolin innig vermischt. Man erhält auf diese Weise ein Stäubemittel, das 5 Gew.-% des Wirkstoffs enthält.
II. 30 Gew.-Teile einer erfindungsgemäßen Verbindung werden mit einer Mischung aus 92 Gew.-Teilen pulverförmigem Kieselsäuregel und8 Gew.-Teilen Paraffinöl, das auf die Oberfläche dieses Kieselsäuregels gesprüht wurde, innig vermischt. Man erhält auf diese weise eine Aufbereitung des Wirkstoffs mit guter Haftfähigkeit (Wirkstoffgehalt 23 Gew.-%).
III. 10 Gew.-Teile einer erfindungsgemäßen Verbindung werden in einer Mischung gelöst, die aus 90 Gew.-Teilen Xylol, 6 Gew.-Teilen des Anlagerungsproduktes von 8 bis 10 Mol Ethylenoxid an 1Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.-%).
IV. 20 Gew.-Teile einer erfindungsgemäßen Verbindung werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5Gew.-Teilen des Anlagerungsproduktes von 40 Mol Ethylenoxid an 1 Mol Ricinusöl besteht (Wirkstoffgehalt 16 Gew.-%).
V. 80 Gew.-Teile einer erfindungsgemäßen Verbindung werden mit 3 Gew.-Teilen des Natriumsalzes der Diisobutylnaphthalinalpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.-%).
VI. Man vermischt 90 Gew.-Teile einer erfindungsgemäßen Verbindung mit 10 Gew.-Teilen N-Methyl-α-pyrrolidon und erhält eine Lösung, die zur Anwendung in Form kleinster Tropfen geeignet ist (Wirkstoffgehalt 90 Gew.-%).
VII. 20 Gew.-Teile einer erfindungsgemäßen Verbindung werden in einer Mischung gelöst, die aus 40 Gew.-Teilen Cyclohexanon, 30Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält maneine wäßrige Dispersion, die 0,02 Gew.-% des Wirkstoffs enthält.
VIII. 20 Gew.-Teile einer erfindungsgemäßen Verbindung werden mit 3 Gew.-Teilen des Matriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20000 Gew.-Teilen Wasser erhält man eine Spritzbrühe, die 0,1 Gew.-% des Wirkstoffs enthält.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Wirkstoffe können zu den erfindungsgemäßen Mitteln im Gewichtsverhältnis 1:10 bis 10:1 zugemischt werden.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt.

### Beispiel 1: Herstellung von 2-(4'-Methoxyiminoeth-1''-yl-phenoxy)phenylglyoxylsäuremethylester-O-methyloxim (Tabelle Nr. 13)

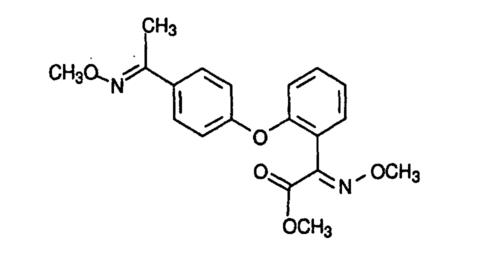

a) 2-(4'-Methoxyiminoeth-1''-yl-phenoxy)phenylglyoxylsäuremethylester
   Zu einer Suspension von 1,4 g Natriumhydrid (95%-ig) in 15 ml Dimethylsulfoxid wurde bei ca. 25°C eine Lösung aus 8,25 g 4-Hydroxyacetophenon-O-methyloxim in 80 ml Dimethylsulfoxid zugetropft und anschließend für 30 Minuten im Ultraschallbad gerührt. Bei ca. 25°C wurde eine Lösung von 9,1 g 2-Fluorphenylglyoxylsäuremethylester in 50 ml Dimethylsulfoxid zugetropft. Nach ca. 24 h bei ca. 25°C wurde die Reaktionsmischung mit ca. 1,2 1 Wasser versetzt. Die so erhaltene Mischung wurde mit tert.-Butyl-methylether extrahiert. Die vereinigten organischen Phasen wurden mit Wasser gewaschen und anschließend getrocknet. Nach Enfernen des Lösungsmittels und chromatographischer Reinigung (Kieselgel mit Cyclohexan/ tert.-Butyl-methylether 5:1 als Laufmittel) erhielt man 10,5g des Produkts als helles Harz.
   - IR [cm⁻¹]:: 1745, 1684, 1599, 1507, 1478, 1456, 1262, 1234, 1205, 1050.
b) Eine Mischung aus 2,3 g des Produkts aus 1a), 0,92 g Methoxyaminhydrochlorid, 0,87 g Pyridin und 30 ml Methanol wurdei für 2 h auf 60°C erhitzt. Nach dem Abkühlen auf ca. 25°C wurde eingeengt. Der verbleibende Rückstand wurde in tert.-Butyl-methylether aufgenommen. Aus der organischen Phase er ) hielt man nach Waschen mit Wasser, Trocknen und Entfernen des Lösungsmittels das Rohprodukt als E/Z-Isomerengemisch. Zur Isomerisierung wurde es in ca. 20 ml Methanol gelöst un< es wurde bei ca. 5 - 10°C Chlorwasserstoff bis zur Sättigung eingegast. Es wurde ca. 12 h bei ca. 25°C gerührt. Nach dem Entfernen des Lösungsmittels wurde der verbleibende Rückstand in tert.-Butyl-methylether aufgenommen. Aus der organischen Phase erhielt man nach.Waschen mit Wasser, Trocknen und Entfernen des Lösungsmittels das isomerisierte Rohprodukt, welches durch Chromatographie an Kieselgel mit Cyclohexan/ tert.-Butyl-methylether 5:1 als Laufmittel aufgereinigt wurde. Man erhielt so insgesamt 1,2g des Produkts (E,E-Isomer) als farblosen Feststoff.
   Fp.: 89 - 91°C.

### Beispiel 2: Herstellung von 2-(4'-Methoxyiminoeth-1''-yl-phenoxy)phenylglyoxylsäuremethylamid-O-methyloxim (Tabelle Nr. 12)

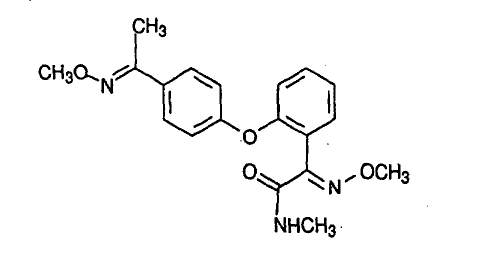

Eine Mischung aus 0,7 g des Produkts aus Beispiel 5, 1,55 g Methylamin (40%-ige wässrige Lösung) und 15 ml Tetrahydrofuran wurde 2 h bei ca. 50°C gerührt. Nach dem Abkühlen wurde das Lösungsmittel unter vermindertem Druck entfernt. Der verbleibende Rückstand wurde in tert.-Butyl-methylether aufgenommen. Aus der organischen Phase erhielt man nach Waschen mit Wasser, Trocknen und Entfernen des Lösungsmittels 0,65 g des Produkts als farblosen Feststoff.
Fp.: 105 - 107°C.

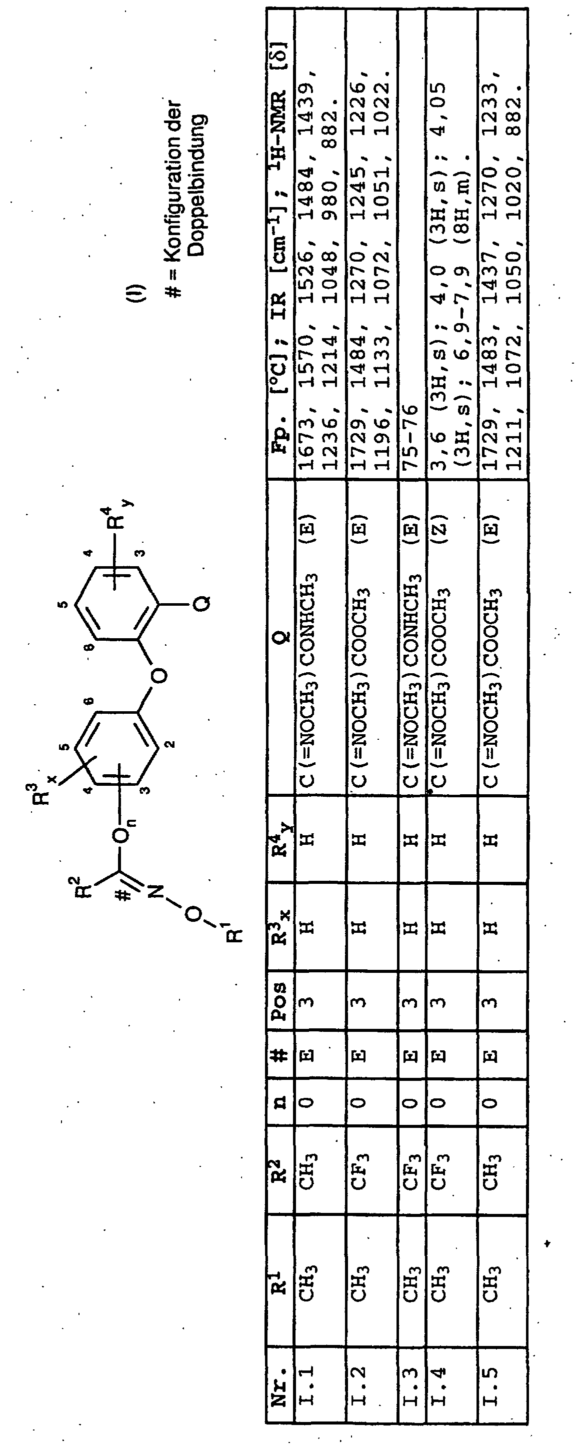

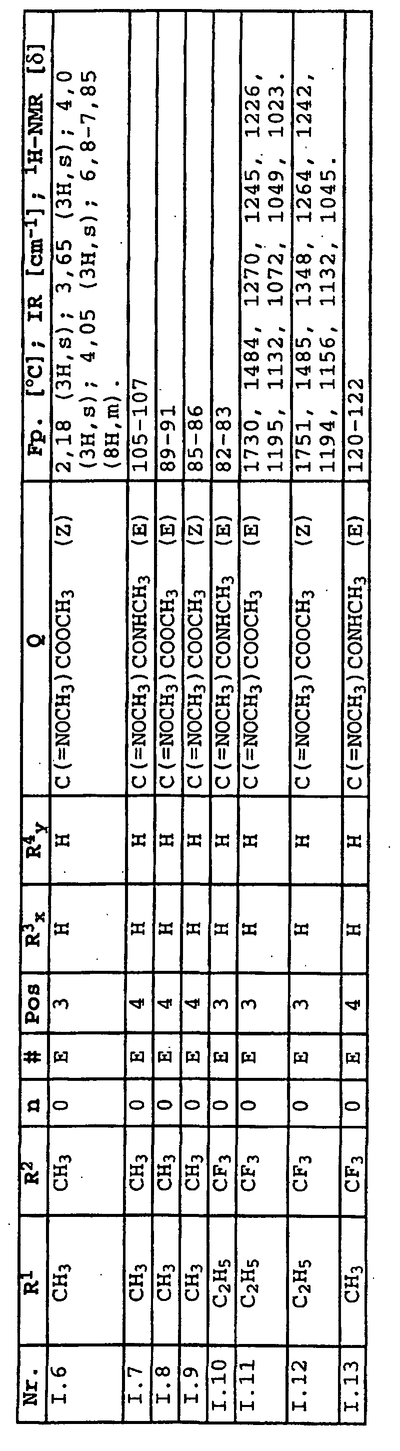

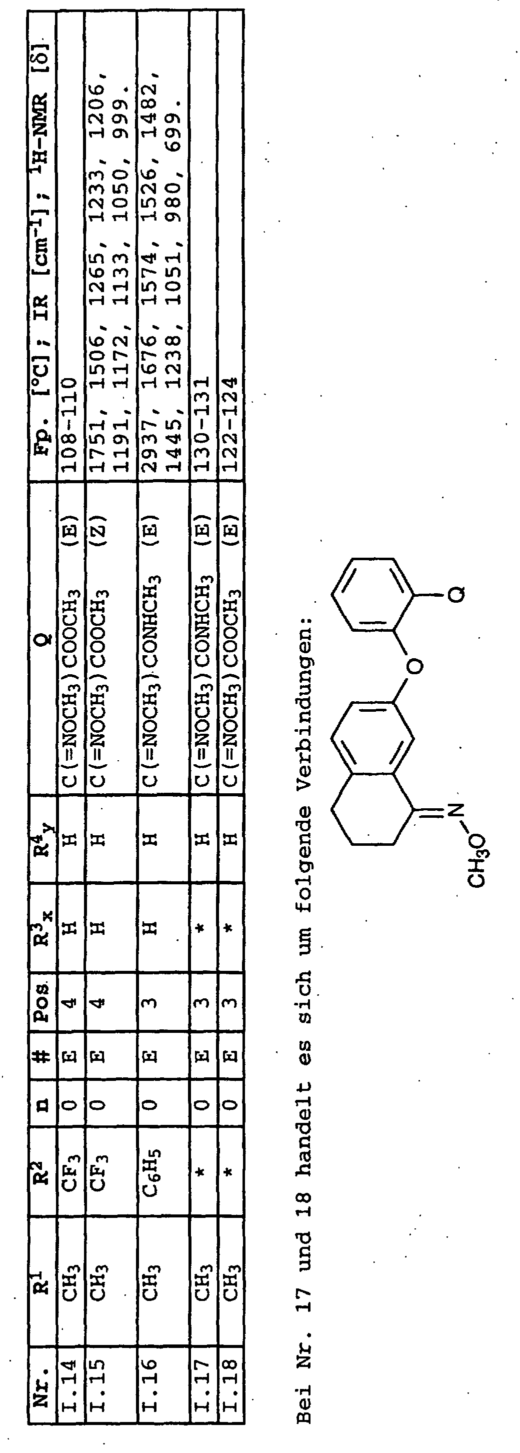

### Beispiele für die Wirkung gegen Schadpilze

Die fungizide Wirkung der Verbindungen der allgemeinen Formel I ließ sich durch die folgenden Versuche zeigen:

Die Wirkstoffe wurden als 20%-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole) aufbereitet und entsprechend der gewünschten Konzentration mit Wasser verdünnt.

### Wirkung gegen Pyricularia oryzae (Reisbrand)

Reiskeimlinge (Sorte: "Tai Nong 67") wurden mit der Wirkstoffaufbereitung tropfnaß gespritzt (Aufwandmenge: 63 ppm). Nach 24 Stunden wurden die Pflanzen mit einer wäßrigen Sporensuspension des Pilzes *Pyricularia oryzae* besprüht und 6 Tage bei 22-24°C bei einer relativen Luftfeuchtigkeit von 95-99 % bewahrt. Die Beurteilung erfolgte visuell.

In diesem Test zeigten die mit den Verbindungen I.1, I.2, I.3, I.4, I.5, I.6, I.10, I.11 und I.16 behandelten Pflanzen einen Befall von 15% und weniger während die unbehandelten Pflanzen zu 75% befallen waren.

### Wirkung gegen Erysiphe graminis var. tritici (Weizenmehltau)

Blätter von Weizenkeimlingen (Sorte "Frühgold") wurden zunächst mit der wäßrigen Aufbereitung der Wirkstoffe behandelt (Aufwandmenge: 250 ppm). Nach ca. 24h wurden die Pflanzen mit Sporen des Weizenmehltaus *(Erysiphe graminis var. tritici*) bestäubt. Die so behandelten Pflanzen wurden anschließend für 7 Tage bei 20-22°C und einer relativen Luftfeuchtigkeit von 75-80% inkubiert. Anschließend wurde das Ausmaß der Pilzentwicklung ermittlelt.

In diesem Test zeigten die mit den Verbindungen I.1, I.2, I.3, I.4, I.5, I.6, I.7, I.8, I.9, I.10, I.11, I.12, I.13, I.14, I.16 und I.17 behandelten Pflanzen einen Befall von 15% und weniger während die unbehandelten Pflanzen zu 75% befallen waren.

### Wirkung gegen Plasmopara viticola (Rebenperonospora)

Topfreben (Sorte: "Müller Thurgau") wurden mit der Wirkstoffaufbereitung tropfnaß gespritzt (Aufwandmenge: 250 ppm). Nach 8 Tagen wurden die Pflanzen mit einer Zoosporenaufschwemmung des Pilzes *Plasmopara viticola* besprüht und 5 Tage bei 20-30°C bei hoher Luftfeuchtigkeit aufbewahrt. Vor der Beurteilung wurden die Pflanzen danach für 16h bei hoher Luftfeuchtigkeit aufbewahrt. Die Auswertung erfolgte visuell.

In diesem Test zeigten die mit den Verbindungen I.I, I.2, I.3, I.4, I.5, I.6, I.10, I.12, I.14, I.16, I.17 und I.18 behandelten Pflanzen einen Befall von 15% und weniger während die unbehandelten Pflanzen zu 80% befallen waren.

### Beispiele für die Wirkung gegen tierische Schädlinge

Die Wirkung der Verbindungen der allgemeinen Formel I gegen tierische Schädlinge ließ sich durch folgende Versuche zeigen:
Die Wirkstoffe wurden
   a. als 0,1%-ige Lösung in Aceton oder
   b. als 10%-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole)
   aufbereitet und entsprechend der gewünschten Konzentration mit Aceton im Fall von a. bzw. mit Wasser im Fall von b. verdünnt.

Nach Abschluß der Versuche wurde die jeweils niedrigste Konzentration ermittelt, bei der die Verbindungen im Vergleich zu unbehandelten Kontrollversuchen noch eine 80 bis 100%-ige Hemmung bzw. Mortalität hervorriefen (Wirkschwelle bzw. Minimalkonzentration).

### Nephotettix cincticeps (Grüne Reiszikade), Kontaktwirkung

Rundfilter wurden mit der wäßrigen Wirkstoffaufbereitung behandelt und anschließend mit 5 adulten Zikaden belegt. Nach 24h wurde die Mortalität beurteilt.

In diesem Test zeigten die erfindungsgemäßen Verbindungen I.2, I.10 und I.11 Wirkschwellen von 0,2 mg.

## Patentansprüche

1. Diphenylether der Formel I, 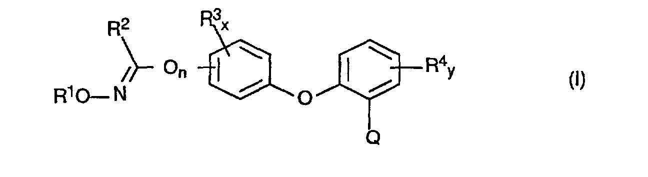 sowie deren Salze und N-Oxide, in denen die Substituenten und Indices die folgende Bedeutung haben:
Q C(CO₂CH₃)=NOCH₃ oder C(CONHCH₃)=NOCH₃;
n 0 oder 1;
R¹ Wasserstoff oder eine der folgenden Gruppen: ggf. subst. Alkyl, Alkenyl, Alkinyl, Cycloalkyl, Heterocyclyl, Aryl oder Heteroaryl;
R² Wasserstoff oder eine der folgenden Gruppen: ggf. subst. Alkyl, Alkenyl, Alkinyl, Cycloalkyl, Heterocyclyl, Aryl oder Heteroaryl, wobei diese Gruppen direkt (über ein Kohlenstoff-Atom) oder über ein Sauerstoff-, Schwefeloder Stickstoff-Atom an das Gerüst gebunden sind;
x 0, 1 oder 2, wobei die Reste R³ verschieden sein können, wenn x für 2 steht;
R³ Cyano, Nitro, Halogen oder eine der folgenden Gruppen: ggf. subst. Alkyl, Alkenyl, Alkinyl, Cycloalkyl, Heterocyclyl, Aryl oder Heteroaryl, wobei diese Gruppen direkt (über ein Kohlenstoff-Atom) oder über ein Sauerstoff-, Schwefel- oder Stickstoff-Atom an das Gerüst gebunden sind oder
R² und ein zur Gruppe Oₙ-C(R²)=NOR¹ benachbarter Substituent R³ C₂-C₃-Alkylen, welches durch ein Sauerstoff- oder Schwefelatom unterbrochen sein kann oder über ein Sauerstoff- oder Schwefelatom an den Phenylring gebunden sein kann;
y 0, 1, 2 oder 3, wobei die Reste R⁴ verschieden sein können, wenn y für 2 oder 3 steht;
R⁴ Cyano, Halogen, C₁-C₄-Alkyl, C₁-C₄-Halogenalkyl oder C₁-C₄-Alkoxy.

2. Verfahren zur Herstellung der Verbindungen I gemäß Anspruch 1, in denen n für 0 steht, **dadurch gekennzeichnet, daß** man ein Phenol der Formel IIa 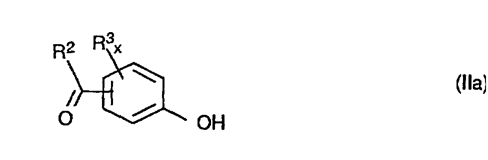 in einem inerten Lösungsmittel mit einem Halogenbenzol der Formel IIIa 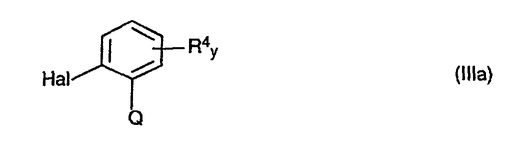 in der Hal für ein Halogenatom steht, in den entsprechenden Ether der Formel IVa 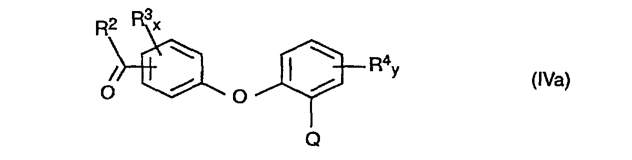 überführt und IVa anschließend mit einem O-substituierten Hydroxylamin (R¹-O-NH₂) oder dessen Salz zu I umsetzt.

3. Verfahren zur Herstellung der Verbindungen I gemäß Anspruch 1, in denen n für 0 steht, **dadurch gekennzeichnet, daß** man ein Phenol der Formel IIa gemäß Anspruch 2 mit einem O-substituierten Hydroxylamin (R¹-O-NH₂) oder dessen Salz in die entsprechende Verbindung der Formel IIb 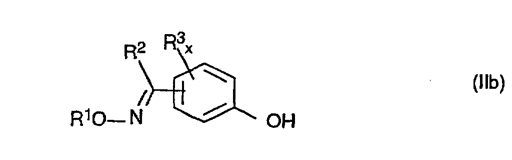 überführt und IIb anschließend in einem inerten Lösungsmittel mit einem Fluorbenzol der Formel IIIa gemäß Anspruch 2, in der Hal Fluor bedeutet, zu I umsetzt.

4. Verfahren zur Herstellung der Verbindungen IVa gemäß Anspruch 2, in denen n für 0 steht, **dadurch gekennzeichnet, daß** man einen Ether der Formel IVb 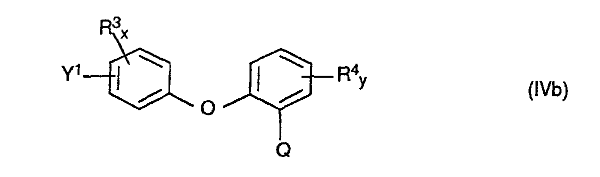 in der Y¹ für ein Halogenatom steht, entweder
a) in Gegenwart einer metallorganischen Base in einem inerten Lösungsmittel mit einer aktivierten Carbonsäure der Formel Va 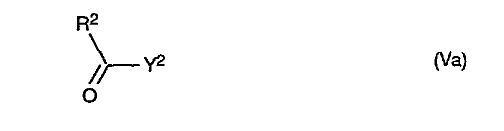 in der Y² für ein Halogenatom, einen Amid- oder einen Esterrest steht, oder einem entsprechenden Cyanid R²-C≡N, oder
b) in einem inerten Lösungsmittel mit einer Zinn-organischen Verbindung der Formel VI 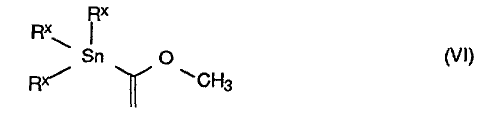 in der die Reste R^{x} voneinander unabhängig für Alkyl stehen,
umsetzt.

5. Verfahren zur Herstellung der Verbindungen I gemäß Anspruch 1, in denen n für 1 steht, **dadurch gekennzeichnet, daß** man einen Diphenylether der Formel IVc 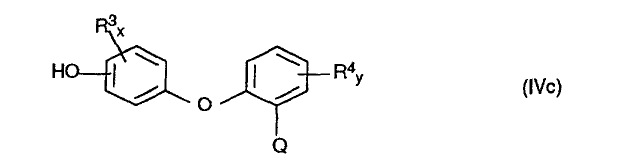 in einem inerten Lösungsmittel in Gegenwart von Salzsäure mit einem Cyanid R²-C≡N in das Hydroxamsäureester-chlorid VIIc 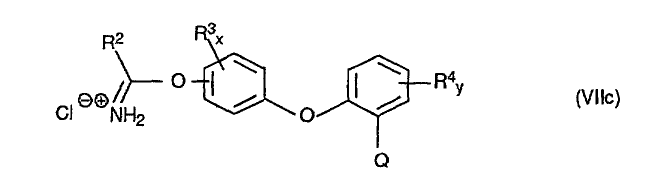 überführt und VIIc anschließend mit einem O-substituierten Hydroxylamin (R¹-ONH₂) oder dessen Salz zu I umsetzt.

6. Verfahren zur Herstellung der Verbindungen I gemäß Anspruch 1, in denen n für 0 und Q für C(CO₂CH₃)=NOCH₃ steht, **dadurch gekennzeichnet, daß** man ein Phenol der Formel IIa gemäß Anspruch 2 in einem inerten Lösungsmittel mit einem Halogenbenzol der Formel IIIb 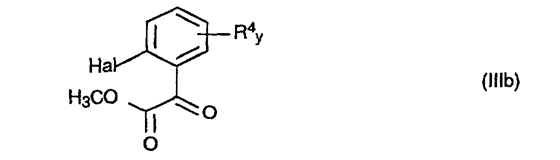 in der Hal für ein Halogenatom steht, in den entsprechenden Ether der Formel VIIa 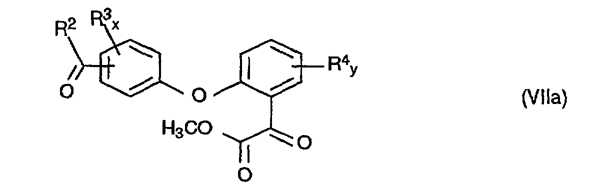 überführt, VIIa anschließend mit einem O-substituierten Hydroxylamin (R¹-ONH₂) oder dessen Salz zu VIIb 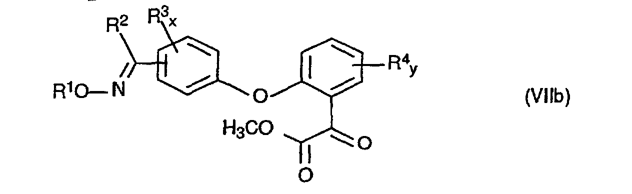 umsetzt und VIIb mit O-Methylhydroxylamin (CH₃-ONH₂) oder dessen Salz umsetzt.

7. Verfahren zur Herstellung der Verbindungen VIIb gemäß Anspruch 6, **dadurch gekennzeichnet, daß** man eine Verbindung der Formel IIb gemäß Anspruch 3 mit einem Halogenbenzol der Formel IIIb gemäß Anspruch 6 umsetzt.

8. Verfahren zur Herstellung der Verbindungen I gemäß Anspruch 1, in denen n für 0 und Q für C(CONHCH₃)=NOCH₃ steht, **dadurch gekennzeichnet, daß** man eine Verbindung der Formel IIb gemäß Anspruch 3 zunächst in einem inerten Lösungsmittel mit einem Halogenbenzol der Formel IIIc 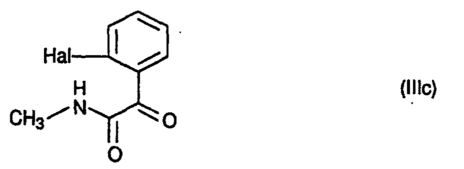 in der Hal für ein Halogenatom steht, in den entsprechenden Ether der Formel VIId 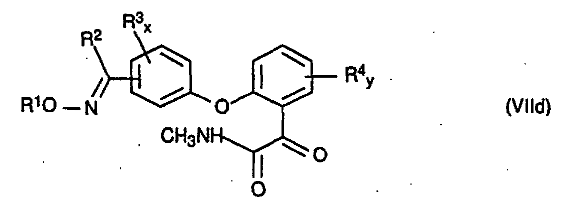 überführt und VIId mit O-Methylhydroxylamin (CH₃O-NH₂) oder dessen Salz umsetzt.

9. Zur Bekämpfung von Schädlingen oder Schadpilzen geeignetes Mittel, enthaltend einen festen oder flüssigen Trägerstoff und eine Verbindung der allgemeinen Formel I gemäß Anspruch 1.

10. Verwendung der Verbindungen I gemäß Anspruch 1 zur Herstellung eines zur Bekämpfung von Schädlingen oder Schadpilzen geeigneten Mittels.

11. Verfahren zur Bekämpfung von Schadpilzen, **dadurch gekennzeichnet, daß** man die Pilze oder die vor Pilzbefall zu schützenden Materialien, Pflanzen, den Boden oder Saatgüter mit einer wirksamen Menge einer Verbindung der allgemeinen Formel I gemäß Anspruch 1 behandelt.

12. Verfahren zur Bekämpfung von Schädlingen, **dadurch gekennzeichnet, daß** man die Schädlinge oder die vor ihnen zu schützenden Materialien, Pflanzen, den Boden oder Saatgüter mit einer wirksamen Menge einer Verbindung der allgemeinen Formel I gemäß Anspruch 1 behandelt.

## Claims

1. A diphenyl ether of the formula I, 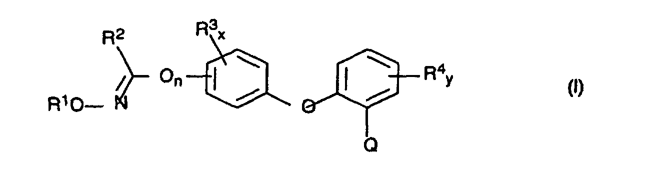 or a salt or N-oxide thereof where the substituents and the indices have the following meanings:
Q is C(CO₂CH₃)=NOCH₃ or C(CONHCH₃)=NOCH₃;
n is 0 or 1;
R¹ is hydrogen or one of the following groups: unsubstituted or substituted alkyl, alkenyl, alkynyl, cycloalkyl, heterocyclyl, aryl or heteroaryl;
R² is hydrogen or one of the following groups: unsubstituted or substituted alkyl, alkenyl, alkynyl, cycloalkyl, heterocyclyl, aryl or heteroaryl, it being possible for these groups to be linked to the skeleton directly (via a carbon atom) or via an oxygen, sulfur or nitrogen atom;
x is 0, 1 or 2, it being possible for the radicals R³ to be different if x is 2;
R³ is cyano, nitro, halogen or one of the following groups: unsubstituted or substituted alkyl, alkenyl, alkynyl, cycloalkyl, heterocyclyl, aryl or heteroaryl, it being possible for these groups to be linked to the skeleton directly (via a carbon atom) or via an oxygen, sulfur or nitrogen atom or
R² and a substituent R³ which is adjacent to the group Oₙ-C(R²)=NOR¹ is C₂-C₃-alkylene which can be interrupted by an oxygen or sulfur atom or can be linked to the phenyl ring via an oxygen or sulfur atom;
y is 0, 1, 2 or 3, it being possible for the radicals R⁴ to be different if y is 2 or 3;
R⁴ is cyano, halogen, C₁-C₄-alkyl, C₁-C₄-haloalkyl or C₁-C₄-alkoxy.

2. A process for the preparation of a compound I as claimed in claim 1 where n is 0, which comprises converting a phenol of the formula IIa 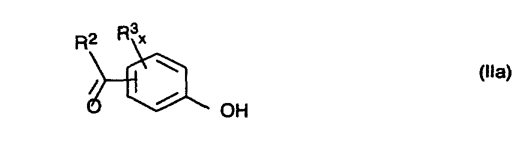 with a halobenzene of the formula IIIa 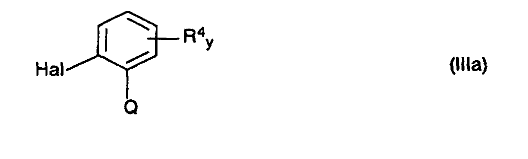 where Hal is a halogen atom in an inert solvent to give the corresponding ether of the formula IVa 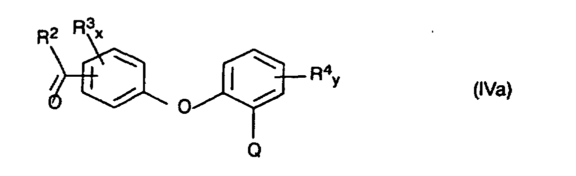 and subsequently reacting IVa with an O-substituted hydroxylamine (R¹-O-NH₂) or with a salt thereof to give I.

3. A process for the preparation of a compound I as claimed in claim 1 where n is 0, which comprises converting a phenol of the formula IIa as set forth in claim 2 with an O-substituted hydroxylamine (R¹-O-NH₂) or with a salt thereof to give the corresponding compound of the formula IIb 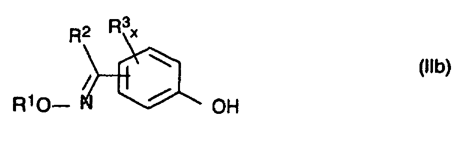 and subsequently reacting IIb with a fluorobenzene of the formula IIIa as set forth in claim 2 where Hal is fluorine in an inert solvent to give I.

4. A process for the preparation of a compound IVa as set forth in claim 2 where n is 0, which comprises reacting an ether of the formula IVb 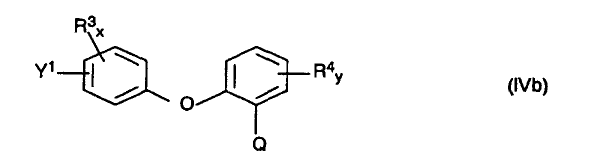 where Y¹ is a halogen atom either
a) with an activated carboxylic acid of the formula Va 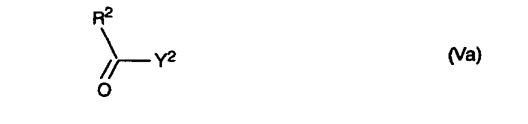 where Y² is a halogen atom, an amide or an ester radical, or with a corresponding cyanide R²-C≡N, in an inert solvent in the presence of an organometallic base, or
b) with an organotin compound of the formula VI 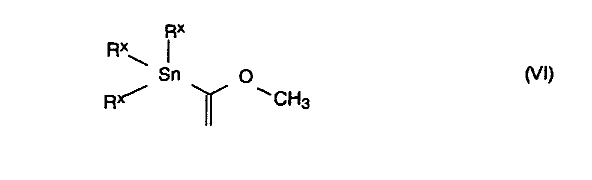 where the radicals R^{x} independently of one another are alkyl,
in an inert solvent.

5. A process for the preparation of a compound I as claimed in claim 1 where n is 1, which comprises converting a diphenyl ether of the formula IVc 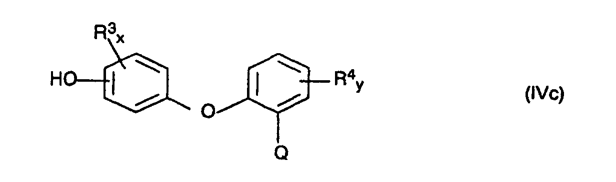 with a cyanide R²-C≡N in an inert solvent in the presence of hydrochloric acid to give the chlorohydroxamate VIIc 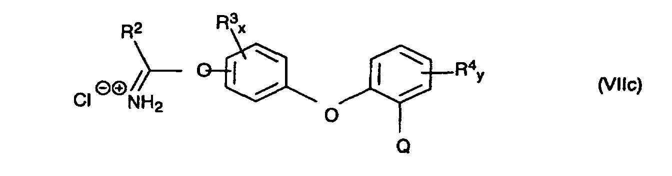 and subsequently reacting VIIc with an O-substituted hydroxylamine (R¹-ONH₂) or with a salt thereof to give I.

6. A process for the preparation of a compound I as claimed in claim 1 where n is 0 and Q is C(CO₂CH₃)=NOCH₃, which comprises converting a phenol of the formula IIa as set forth in claim 2 with a halobenzene of the formula IIIb 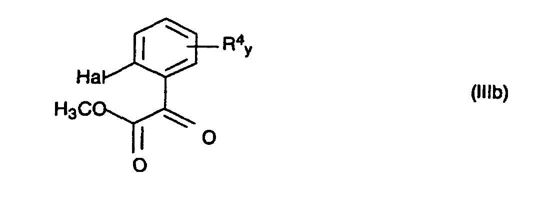 where Hal is a halogen atom in an inert solvent to give the corresponding ether of the formula VIIa 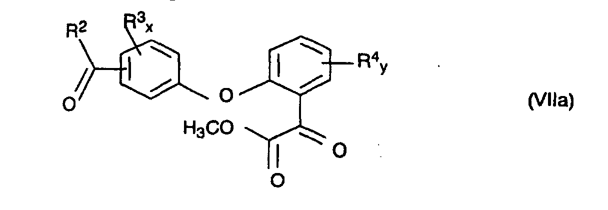 subsequently reacting VIIa with an O-substituted hydroxylamine (R¹-ONH₂) or with a salt thereof to give VIIb 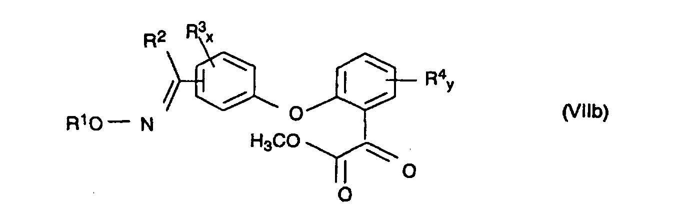 and reacting VIIb with O-methylhyd oxy amine (CH₃-ONH₂) or with a salt thereof.

7. A process for the preparation of a compound VIIb as set forth in claim 6, which comprises reacting a compound of the formula IIb as set forth in claim 3 with a halobenzene of the formula IIIb as set forth in claim 6.

8. A process for the preparation of a compound I as claimed in claim 1 where n is 0 and Q is C(CONHCH₃)=NOCH_{3,} which comprises first converting a compound of the formula IIb as set forth in claim 3 with a halobenzene of the formula IIIc 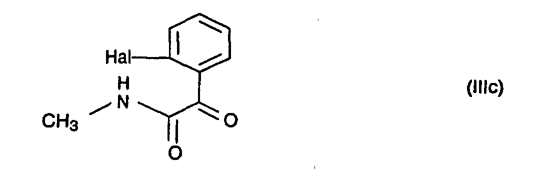 where Hal is a halogen atom i.. an inert solvent to give the corresponding ether of the formula VIId 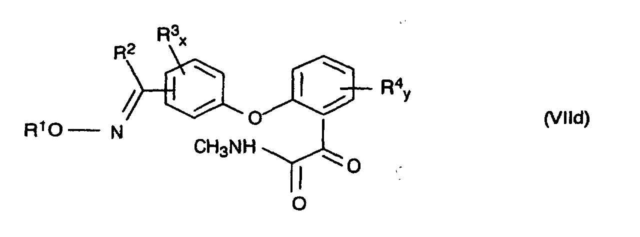 and reacting VIId with O-methylhydroxylamine (CH₃O-NH₂) or the salt thereof.

9. A composition which is suitable for controlling pests or harmful fungi, comprising a solid or liquid carrier and a compound of the general formula I as claimed in claim 1.

10. The use of a compound I as claimed in claim 1 for the preparation of a composition which is suitable for controlling pests or harmful fungi.

11. A method of controlling harmful fungi, which comprises treating the fungi, or the materials, plants, the soil or seeds to be protected against fungal infection, with an effective amount of a compound of the general formula I as claimed in claim 1.

12. A method of controlling pests, which comprises treating the pests, or the materials, plants, the soil or seeds to be protected against them, with an effective amount of a compound of the general formula I as claimed in claim 1.

## Revendications

1. Ethers diphényliques de formule I 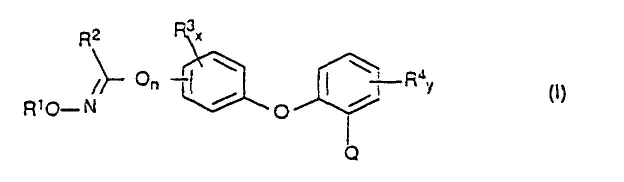 leurs sels et N-oxydes, les symboles et indices de la formule ayant les significations suivantes :
Q : C(CO₂CH₃)=NOCH₃ ou C(CONHCH₃)=NOCH₃ ;
n : 0 ou 1 ;
R¹ : l'hydrogène ou l'un des groupes suivants : alkyle, alcényle, alcynyle, cycloalkyle, hétérocyclyle, aryle ou hétéroaryle éventuellement substitué ;
R² : l'hydrogène ou l'un des groupes suivants : alkyle, alcényle, alcynyle, cycloalkyle, hétérocyclyle, aryle ou hétéroaryle éventuellement substitué, ces groupes pouvant être reliés au squelette directement (par l'intermédiaire d'un atome de carbone) ou par l'intermédiaire d'un atome d'oxygène, de soufre ou d'azote ;
x : 0, 1 ou 2, les symboles R³ pouvant avoir des significations différentes lorsque x est égal à 2 ;
R³ : un groupe cyano, nitro, un halogène ou l'un des groupes suivants : alkyle, alcényle, alcynyle, cycloalkyle, hétérocyclyle, aryle ou hétéroaryle éventuellement substitué, ces groupes pouvant être reliés au squelette directement (par l'intermédiaire d'un atome de carbone) ou par l'intermédiaire d'un atome d'oxygène, de soufre ou d'azote, ou bien
R² et un substituant R³ voisin du groupe On-C(R²)=NOR¹ représentent un groupe alkylène en C2-C3 qui peut être interrompu par un atome d'oxygène ou de soufre ou peut être relié au cycle phényle par un atome d'oxygène ou de soufre ;
y : 0, 1, 2 ou 3, les symboles R⁴ pouvant avoir des significations différentes lorsque y est égal à 2 ou 3 ;
R⁴ : un groupe cyano, un halogène, un groupe alkyle en C1-C4, halogénoalkyle en C1-C4 ou alcoxy en C1-C4.

2. Procédé pour la préparation des composés I selon la revendication 1 pour lesquels n est égal à 0, **caractérisé par le fait que** l'on convertit un phénol de formule IIa 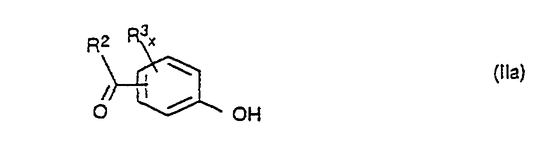 dans un solvant inerte, à l'aide d'un halogénobenzène de formule IIIa 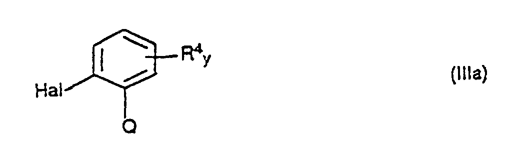 dans laquelle Hal représente un atome d'halogène, en l'éther correspondant de formule IVa 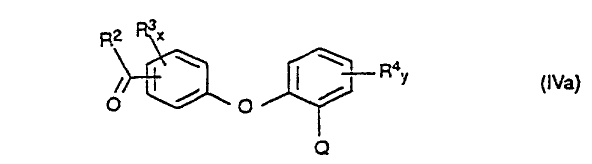 qu'on convertit ensuite en I à l'aide d'une hydroxylamine substituée à l'oxygène (R¹-O-NH₂) ou de l'un de ses sels.

3. Procédé pour la préparation des composés I selon la revendication 1 pour lesquels n est égal à 0, **caractérisé par le fait que** l'on convertit un phénol de formule IIa selon la revendication 2, à l'aide d'une hydroxylamine substituée à l'oxygène (R¹-O-NH₂) ou de l'un de ses sels, en le composé correspondant de formule IIb 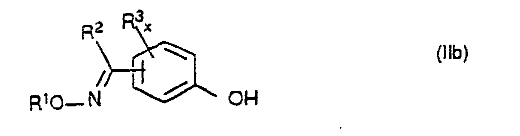 qu'on convertit ensuite en I dans un solvant inerte à l'aide d'un fluorobenzène de formule IIIa selon la revendication 2 dans laquelle Hal représente le fluor.

4. Procédé pour la préparation des composés IVa selon la revendication 2 pour lesquels n est égal à 0, **caractérisé par le fait que**, partant d'un éther de formule IVb 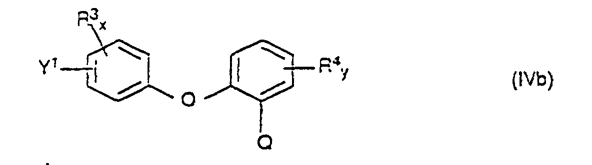 dans laquelle Y¹ représente un atome d'halogène,
a) ou bien on le fait réagir en présence d'une base organométallique, dans un solvant inerte, avec un acide carboxylique activé de formule Va 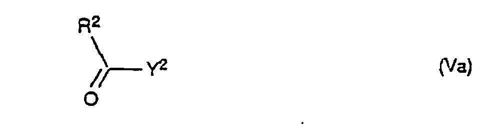 dans laquelle Y² représente un atome d'halogène, un radical d'amide ou un radical d'ester, ou avec un cyanure correspondant R²-C≡N,
b) ou bien on le fait réagir dans un solvant inerte avec un dérivé organique de l'étain répondant à la formule VI 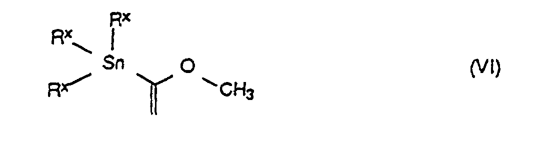
dans laquelle les symboles R^{x} représentent chacun, indépendamment les uns des autres, un groupe alkyle.

5. Procédé pour la préparation des composés I selon la revendication 1 pour lesquels n est égal à 1, **caractérisé par le fait que** l'on convertit un éther diphénylique de formule IVc 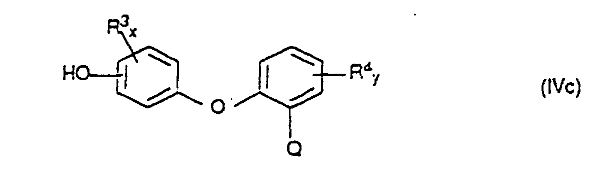 dans un solvant inerte, en présence d'acide chlorhydrique, à l'aide d'un cyanure R²-C≡N en le chlorure d'ester hydroxamique VIIc 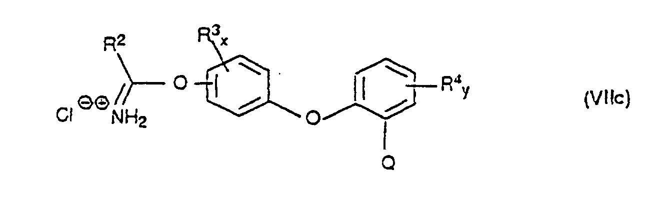 qu'on convertit ensuite en I à l'aide d'une hydroxylamine substituée à l'oxygène (R¹-ONH₂) ou de l'un de ses sels.

6. Procédé pour la préparation des composés I selon la revendication 1 pour lesquels n est égal à 0 et Q représente C(CO₂CH₃)=NOCH₃, **caractérisé par le fait que** l'on convertit un phénol de formule IIa selon la revendication 2, dans un solvant inerte, à l'aide d'un halogénobenzène de formule IIIb 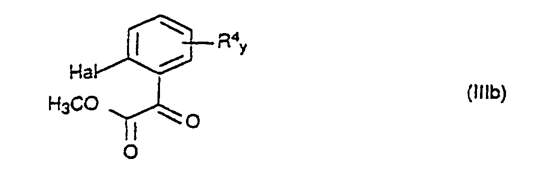 dans laquelle Hal représente un atome d'halogène, en l'éther correspondant de formule VIIa 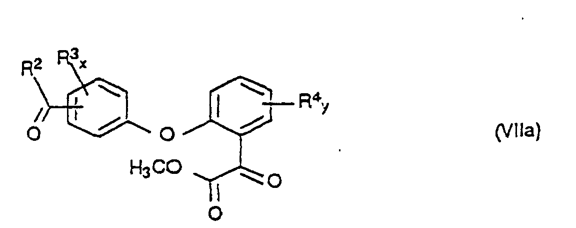 qu'on convertit ensuite, à l'aide d'une hydroxylamine substituée à l'oxygène (R¹-ONH₂) ou de l'un de ses sels, en le composé VIIb 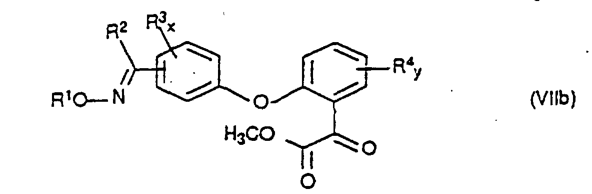 qu'on fait ensuite réagir avec l'O-méthylhydroxylamine (CH₃-ONH₂) ou de l'un de ses sels.

7. Procédé pour la préparation des composés VIIb selon la revendication 6, **caractérisé par le fait que** l'on fait réagir un composé de formule IIb selon la revendication 3 avec un halogénobenzène de formule IIIb selon la revendication 6.

8. Procédé pour la préparation des composés I selon la revendication 1 pour lesquels n est égal à 0 et Q représente C(CONHCH₃)=NOCH₃, **caractérisé par le fait que** l'on convertit d'abord un composé de formule IIb selon la revendication 3, dans un solvant inerte, à l'aide d'un halogénobenzène de formule IIIc 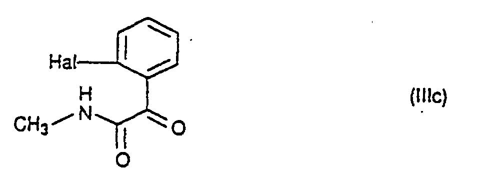 dans laquelle Hal représente un atome d'halogène, en l'éther correspondant de formule VIId 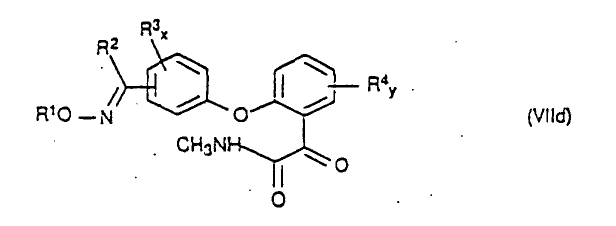 qu'on fait ensuite réagir avec l'O-méthylhydroxylamine (CH₃O-NH₂) ou l'un de ses sels.

9. Produit apte à l'utilisation pour la lutte contre les parasites ou les mycètes nuisibles, contenant un véhicule solide ou liquide et un composé de formule générale I selon la revendication 1.

10. Utilisation des composés I selon la revendication 1 pour la préparation d'un produit apte à l'utilisation pour la lutte contre les parasites ou les mycètes nuisibles.

11. Procédé pour combattre les mycètes nuisibles, **caractérisé par le fait que** l'on traite les mycètes ou les matériaux, végétaux, sols ou semences qu'on veut protéger contre l'attaque par les mycètes par une quantité efficace d'un composé de formule I selon la revendication 1.

12. Procédé pour combattre les parasites, **caractérisé par le fait que** l'on traite les parasites ou les matériaux, végétaux, sols ou semences qu'on veut protéger contre les parasites par une quantité efficace d'un composé de formule générale I selon la revendication 1.
